# EUROPEAN PATENT APPLICATION

(11) **EP 2 422 688 A1**
(43) Date of publication of application: **29.02.2012**
(21) Application number: 11178241.3
(22) Date of filing: 22.08.2011
(51) Int. Cl.: A61B 1/07, A61B 5/00, A61B 1/06, A61B 1/00, A61B 1/005

(54) **Electronic endoscope system**

(30) Priority: 31.08.2010 JP 2010194888
(71) Applicant: Fujifilm Corporation, Minato-ku Tokyo (JP)
(72) Inventor: Saito, Maki, Kanagawa (JP); Iida, Takayuki, Kanagawa (JP); Yasuda, Hiroaki, Kanagawa (JP); Hara, Shoji, Kanagawa (JP)
(74) Representative: Klunker . Schmitt-Nilson . Hirsch

(57) **Abstract**

In an electronic endoscope system (303), the intensity of therapeutic light projected from a therapeutic light unit (62) into a subject body may be lowered by a stop mechanism (72) to avoid halation. With the low intensity therapeutic light and ordinary white light from an illumination light unit (61), a CCD (21) captures an image from an interior of the subject body. From the captured image, an irradiance estimator (303) extracts data about the position and power distribution of irradiation by the therapeutic light. A display control circuit (44) controls a monitor (22) to display the extracted data in combination with the captured image, allowing the operator to adjust the projected position and direction of the therapeutic light to be efficient for photodynamic treatment.

## Description

### BACKGROUND OF THE INVENTION

### 1. Field of the Invention

The present invention relates to an electronic endoscope system that can capture images from interior of a subject body and can also project therapeutic laser light toward an affected part of the subject body.

### 2. Description of the Related Art

In recent medical field, electronic endoscopes are widely used for diagnoses and treatments. The electronic endoscope projects white light or ordinary light into body cavities of a subject or patient to visualize the interior of the subject body through an imaging unit. The images captured under the ordinary light provide a natural view of the interior of the subject body, but it can be difficult to discriminate a lesion like a tumor site from normal tissues in the natural view. In order to clearly visualize affected part, a specific narrowband ray or special light is used to illuminate the interior of the subject body during the imaging. An electronic endoscope system for making photodynamic diagnosis (PDD) is known as one of those endoscope systems which use a special light for identifying tumor cells or the like. Such an electronic endoscope system is also known in the art that can conduct photodynamic therapy (PDT) on the identified lesion or tumor site.

The PDD is a diagnostic method using a photosensitizer that emits fluorescence as it is irradiated with exciting light of a certain wavelength. The photosensitizer is previously injected into a subject body and accumulated in tumor cells of the subject body, so that the location and the size of the tumor site may be determined from the fluorescence emitted from the accumulated photosensitizer. Known as one of typical oncotropic photosensitizers is hematoporphyrin derivative that emits red fluorescence, at a wavelength of 635 nm for example, when it is irradiated with a blue ray of around 405 nm as the exciting light.

It is known in the art that the photosensitizer accumulated in the tumor cells for the PDD will generate reactive oxygen species when it is irradiated with special light of a different wavelength from that of the exciting light. For example, the special light may be a red ray ranging from 630 nm to 680 nm. Because the reactive oxygen species have a cytocidal effect, the tumor cells containing the photosensitizer therein will be extinguished by the reactive oxygen species. The PDT is a treatment utilizing the cytocidal effect of the reactive oxygen species, and the special light for causing the photosensitizer to generate the reactive oxygen species may be called the therapeutic light.

Although it varies depending upon the size of the tumor site and the power of the therapeutic light, the PDT takes ten minutes or so for one session. This treatment time is comparatively long as for endoscopic treatments.

Meanwhile, the therapeutic light for the PDT is projected at a very high intensity in comparison with the ordinary light. Therefore, endoscopic images tend to have so-called halation during the PDT as they are captured while the therapeutic light and the ordinary light are being projected into the subject body. When halation occurs, an image area corresponding to a portion irradiated with the therapeutic light, i.e. a projection spot, and its periphery will be breached or overexposed. Accordingly, it can often be difficult to observe the interior of the subject body during the PDT. Moreover, because the fluorescence to be imaged for the PDD is in a similar wavelength band to the therapeutic light, it is difficult to make the PDD and the PDT concurrently on the same subject. However, it is desirable to image the interior of the subject body continuously during the PDT so that the operator may keep track of the target for irradiation and check the efficiency of this treatment.

JPA Nos. 2006-130183 and 2006-94907 disclose such an electronic endoscope system that enables observing the interior of the subject body under the ordinary light and/or making the PDD while executing the PDT by synchronizing the timing of projection of the different kinds of light appropriately with the driving timing of the imaging device. In the endoscopic system disclosed in JPA 2006-130183, the PDT or projection of the therapeutic light is carried out in the first field of one imaging frame, and the PDD or projection of the exciting light is carried out in the second field of the imaging frame, so that both the PDT and the PDD are carried out in one frame. In the endoscopic system disclosed in JPA 2006-94907, imaging under the ordinary light is carried out in one frame, the PDD is carried out in the next frame, and the PDT is carried out in the frame after the next. Thus the ordinary light, the exciting light and the therapeutic light are cyclically projected into the subject body. The latter prior art also discloses resetting charges accumulated in the imaging device before the end of one frame for the PDT, thereby to suppress the halation.

In order to extinguish a tumor by the PDT, the tumor should be irradiated with a sufficient amount of therapeutic light. The integral dose of the therapeutic light necessary for extinguishing tumor cells depends on the size and other features of the tumor. As a property of the light source, the therapeutic light is projected at a constant intensity or light volume per unit time. Therefore, the requisite treatment time for the PDT may be calculated by the intensity of the therapeutic light and the integral dose of the therapeutic light necessary for accomplishing the treatment of the tumor.

However, depending on the projected position and direction of the therapeutic light onto the tumor site, the tumor site cannot be effectively irradiated with the therapeutic light. If the therapeutic light is not efficiently projected onto the tumor site, the tumor cells may not be completely extinguished due to lack of therapeutic light even while the therapeutic light has been projected for the calculated requisite treatment time. To make sure to extinguish the tumor cells completely, there has conventionally been nothing better than to project the therapeutic light for a longer time than the calculated requisite treatment time. The elongated treatment time will put a greater burden on the subject body.

Since most images captured while the therapeutic light is being projected have halation, it is hard to see from the images with halation whether the therapeutic light is effectively projected onto a tumor site. Although it is possible to capture an image without halation under the ordinary light by interrupting the therapeutic light in one frame and display the image without halation on a monitor, it is impossible to confirm from this ordinary light image whether the tumor site is effectively irradiated with the therapeutic light.

### SUMMARY OF THE INVENTION

The present invention has an object to provide an electronic endoscope system that helps the operator project the therapeutic light efficiently onto a target site.

In an electronic endoscope system having a white light projecting device for projecting white light into an interior of a subject body, a therapeutic light projecting device, and an imaging device, the present invention provides a light volume adjusting device for adjusting the volume of therapeutic light to such a low intensity that no halation can occur in an image captured by the imaging device while white light and the low intensity therapeutic light are being projected into the interior of the subject body; an extracting device for extracting from the captured image data about the position and power of irradiation by the low intensity therapeutic light inside the subject body; and a display control device for controlling a display device to display the data extracted by the extracting device in combination with the image. The imaging device images the interior of the subject body through photoelectric conversion of light received from the interior of the subject body. The therapeutic light projecting device projects the therapeutic light onto a therapeutic target site to be treated inside the subject body. The therapeutic light gives a therapeutic effect on the target site by acting on a photosensitive substance that has been accumulated in the target site.

In a preferred embodiment, the electronic endoscope system has a range designating device for designating a range of interest in the captured image, wherein the extracting device extracts the data about the distribution of irradiance of the low intensity therapeutic light along vertical and horizontal axes of the captured image, these axes intersecting at a center of the range of interest.

The extracting device may preferably extract the data based on pixel levels of one color among pixel levels of the image, the one color corresponding to a wavelength band of the therapeutic light.

The display control device may preferably produce histograms from the extracted data and controls the display device to display the histograms with the image.

In another embodiment, the electronic endoscope system further includes an exciting light projecting device for projecting exciting light into the interior of the subject body, the exciting light causing the photosensitive substance to emit fluorescence; an identifying device for identifying the target site based on a fluorescent light image captured by imaging the fluorescent light that is emitted from the photosensitive substance upon the exciting light being projected into the interior of the subject body. In this embodiment, the range designating device may automatically designate the range of interest to include the target site as identified by the identifying device.

Preferably, the electronic endoscope system further includes a halation detecting device for detecting whether an image frame has halation or not when the image frame has been captured by the imaging device while the therapeutic light is being projected concurrently with the white light into the subject body; and a memory device for storing a plural number of image frames captured by the imaging device. In this embodiment, when the halation detecting device detects that the latest image frame captured by the imaging device has halation, the display control device selects an image frame without halation from among image frames stored in the memory device, to display the selected image frame instead of the latest image frame on the display device.

The halation detecting device determines that one image frame has halation when the number of those pixels having higher levels than a predetermined threshold value is above a predetermined number among pixels constituting the one image frame, and that the one image frame does not have halation when the number of those pixels having higher levels than the predetermined threshold value is not more than the predetermined number.

The electronic endoscope system may further include a control device for controlling the light volume adjusting device to lower the intensity of the therapeutic light to forcibly capture an image frame without halation.

The memory device temporarily stores a constant number of image frames in the order captured, and the control device controls the light volume adjusting device to lower the intensity of the therapeutic light before all image frames stored in the memory device have halation.

The control device may control the light volume adjusting device to lower the intensity of the therapeutic light in the next frame when a predetermined number of successive image frames have been captured with halation, the predetermined number being smaller than the constant number.

In the electronic endoscope system of the present invention, the intensity of therapeutic light projected into a subject body may be lowered to avoid halation. While the low intensity therapeutic light and ordinary white light are being concurrently projected into the subject body, an image is captured from the interior of the subj ect body, and data about the position and power distribution of the therapeutic light is extracted from the captured image. Since the extracted data is displayed in combination with the captured image, the operator can adjust the projected position and direction of the therapeutic light relative to the subject body so as effectively to irradiate a target site such as tumor cells inside the subject body.

Keeping displaying endoscopic images without halation on the monitor during the treatment with the therapeutic light enables the operator to adjust the projected position and direction of the therapeutic light relative to the target site while observing the interior of the subject body. Thus, the operator can project the therapeutic light efficiently onto the target site, saving the total treatment time.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Figure 1 is a diagram illustrating an outer appearance of an electronic endoscope system in accordance with to a first embodiment of the present invention;
Figure 2 is a block diagram illustrating the circuitry of the electronic endoscope system of the first embodiment;
Figure 3 is an explanatory diagram illustrating a stop mechanism;
Figure 4 is a graph showing characteristics of a double-notch filter;
Figure 5 is a flowchart illustrating an operation of the electronic endoscope system;
Figures 6A to 6D are explanatory diagrams illustrating various aspects of images captured by the electronic endoscope system;
Figure 7 is an explanatory diagram illustrating an operation for displaying an image without halation as a substitute for an image with halation;
Figure 8 is an explanatory diagram illustrating an operation for forcibly capturing an image without halation;
Figures 9A and 9B are timing charts showing relationships between the ON-OFF timing of the ordinary light and the therapeutic light and the interval of imaging;
Figure 10 are timing charts of an embodiment where the PDT and the PDD are alternately executed;
Figure 11 is a block diagram illustrating a circuitry of an electronic endoscope system in accordance with another embodiment;
Figures 12A to 12E are explanatory diagrams illustrating an embodiment where an edge of halation zone or a projection spot is displayed as a superimposed image during the PDT;
Figure 13 is an explanatory diagram illustrating another embodiment where an edge of halation zone is displayed as a superimposed image during the PDT;
Figure 14 is a block diagram illustrating a circuitry of an electronic endoscope system in accordance with another embodiment;
Figure 15 is an explanatory diagram illustrating an embodiment for displaying the illuminance distribution of therapeutic light during the PDT;
Figure 16 is an explanatory diagram illustrating positioning of an endoscope tip relative to a tumor site;
Figure 17 is a block diagram illustrating a circuitry of an electronic endoscope system in accordance with another embodiment;
Figure 18 is an explanatory diagram illustrating another embodiment for displaying the irradiance distribution therapeutic light during the PDT;
Figure 19 is an explanatory diagram illustrating still an embodiment for displaying the irradiance distribution therapeutic light during the PDT;
Figure 20 is a block diagram illustrating an electronic endoscope system where laser diodes are used as light sources for all of the ordinary light, the exciting light and the therapeutic light;
Figures 21A and 21B are schematic sectional views illustrating structures of two pairs of light projection units used in the embodiment of Fig.20;
Figure 22 is a face end view of an endoscope having the light projection units; and
Figure 23 is a table showing the wavelengths of exciting light for typical photosensitizers, the wavelengths of fluorescence from the respective photosensitizers, and the wavelength of the therapeutic light for the respective photosensitizers.

### DESCRIPTION OF THE PREFERRED EMBODIMENTS

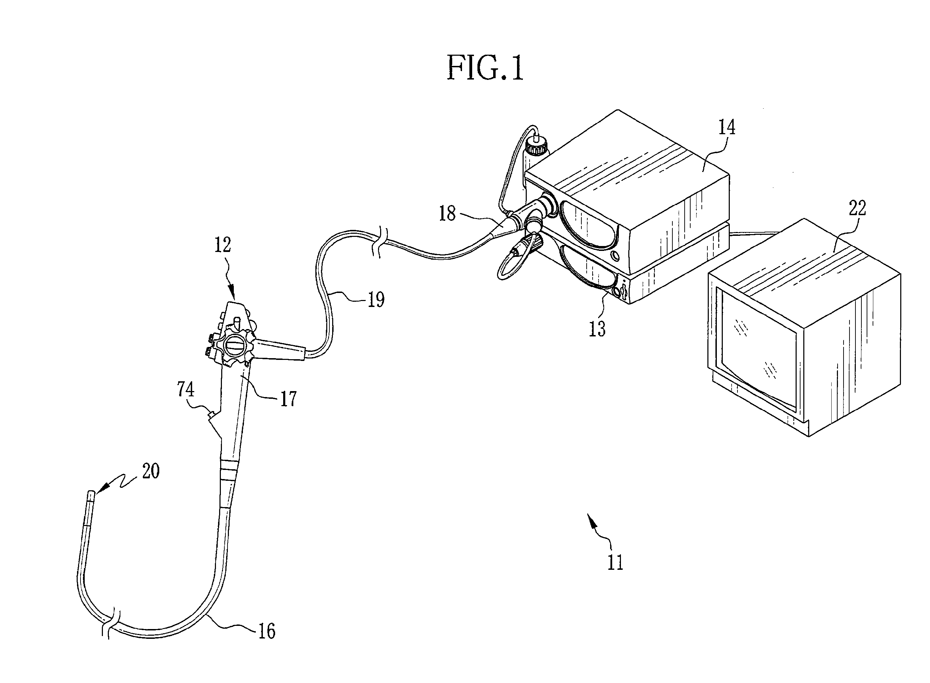

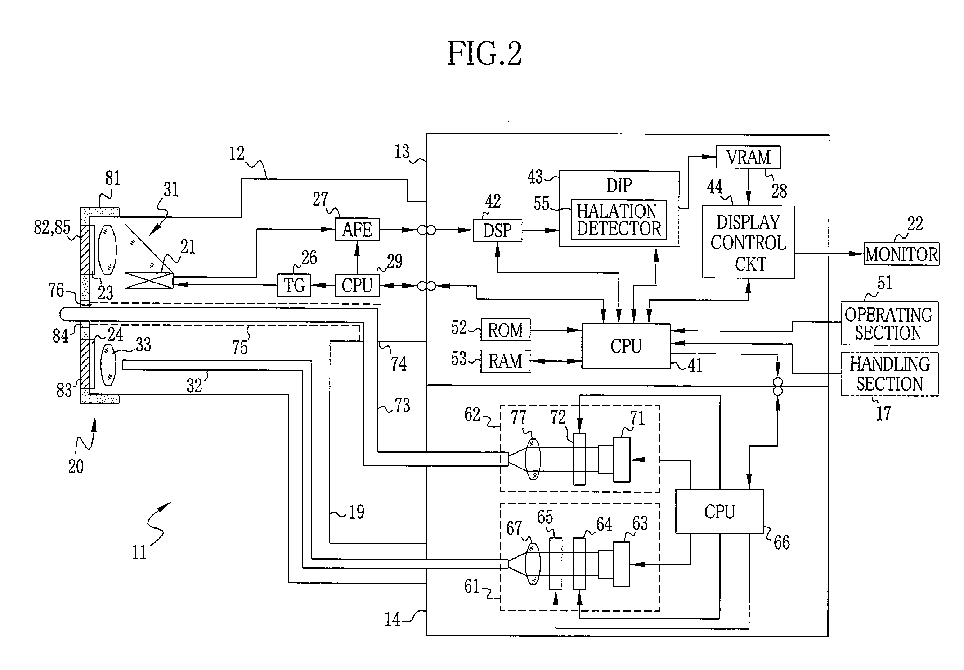

As shown in Fig. 1, an electronic endoscope system 11 according to the first embodiment of the present invention includes an electronic endoscope 12, a processing apparatus 13, and a light source apparatus 14. The electronic endoscope 12 includes a flexible probing portion 16 to be inserted into the body cavity, a handling section 17 coupled to a proximal end of the probing portion 16, a connector 18 connected to the processing apparatus 13 and the light source apparatus 14, and an universal cord 19 connecting the handling section 17 and the connector 18. In a tip 20 of the probing portion 16 is provided a CCD image sensor 21 (see Fig.2) for imaging the interior of the body cavity.

The processing apparatus 13 is electrically coupled to the light source apparatus 14, and supervises the overall operation of the electronic endoscope system 11. Through a cable that is conducted through inside the universal cord 19 and the probing portion 16, the processing apparatus 13 controls power supply to the electronic endoscope 12 and also controls driving the image sensor 21. The processor 13 receives image signals from the image sensor 21 through the cable, and process the image signals to produce image data. Based on the image data produced from the processor 13, images captured by the electronic endoscope 12 may be displayed on a monitor 22 that is connected to the processing apparatus 13 through a cable.

The handling section 17 includes an angle knob for curving the probing portion 16 to orient the tip 20 in any appropriate direction. The handling portion includes other operation members such as an air-jet button for blowing air from the tip 20, a watering button for ejecting water from the tip 20, a release button for recording an observed image as a still image frame, a zooming button for changing the magnification of the images displayed on the monitor 22, and a switching button for switching between an ordinary observation mode wherein the ordinary light is used for illumination and a photodynamic diagnosis (PDD) mode wherein only an exciting light is used for illumination. A tool inlet 74 is provided in a distal part of the handling section 17, allowing insertion of an appropriate therapeutic tool, such as an electric scalpel or a light guide or therapeutic probe for conducting a therapeutic light for the photodynamic therapy (PDT) , into a channel 75 inside the probing portion 16, as shown in Fig.2. The tool inlet 74 is connected through the channel 75 to a tool outlet 76 that is provided in the tip 20.

As shown in Fig.2, the tip 20 is provided with an imaging window 23 and a lightening window 24. A timing generator (TG) 26, an analog signal processing circuit (AFE) 27, VRAM 28, and CPU 29 are disposed in the handling section 17 or in the connector 18 of the universal cord 19. The CCD 21 is located behind the imaging window 23 such that an image of the interior of the subject body is formed on an imaging surface of the CD 21 through an objective lens system 31 consisting of a cluster of lenses and a prism. The lightening window 24 is for projecting illumination light toward the interior of the subject body. The illumination light is supplied from the light source apparatus 14 to the electronic endoscope 12 through a light guide 32 that is conducted through the universal cord 19 and the probing portion 16. Then the illumination light is projected through a projection lens 33 and the lightening window 24 into the subject body.

The CCD 21 has a lot of image sensor pixels that accumulate electronic charges at different levels corresponding to the amounts of light incident on the respective pixels. Thus the CCD 21 converts an optical image of the interior of the subject body, which is formed on the imaging surface of the CCD 21 through the objective lens system 31, into an electronic image signal. The imaging surface consists of a central photo sensor area and an optical black surrounding the photo sensor area. In the photo sensor area, open pixels are arranged in an array, and a color filter consisting of multiple color segments is formed on the pixels. The color filter may for example be a primary color filter (RGB filter) or a complementary color filter (CMY or CMYG filter) having the color segments arranged in the Beyer layout. The optical black is an area consisting of closed pixels which are shielded by a shielding layer. The closed pixels output an image signal corresponding to a dark current noise. Thus, the image signal output from the CCD 21 includes the image signal output from the pixels in the photo sensor area and the image signal output from the optical black pixels. The image signal output from the pixels in the photo sensor area is used to produce the visible image, whereas the image signal output from the optical black pixels is used for dark-current correction of the image signal from the photo sensor area.

The image signal output from the image sensor 21 is an analog signal, which is subjected to a noise reduction process and a gain correction process in the AFE 27, and then converted to a digital signal. Thereafter, the digital image signal is fed to a digital signal processor (DSP) 42 of the processing apparatus 13 through the universal cord 19 and the connector 18.

The TG 26 supplies the CCD 21 with a clock signal. The image sensor 21 conducts imaging and outputs the image signal in accordance with the clock signal from the TG 26. The CPU 29 drives the TG 26 on the basis of an operational signal from a CPU 41 of the processor 13 after the electronic endoscope 12 is connected to the processor 13.

The AFE 27 is constituted of a correlated double sampling circuit (CDS), an automatic gain control circuit (AGC), and an analog-to-digital converter (A/D), which are not shown in the drawings. The CDS processes the image signal from the CCD 21 through correlated double sampling, to eliminate noises that may be caused by driving the CCD 21. The AGC amplifies the image signal after the noise reduction through the CDS. The A/D converts the amplified image signal to a digital image signal of a predetermined bit number. The AFE 27 is controlled by the CPU 29. For example, the CPU 29 controls the amplification rate (gain) of the image signal through the AGC on the basis of an operational signal from the CPU 41 of the processor 13.

The processor 13 includes the CPU 41, the DSP 42, a digital image processor (DIP) 43, a display control circuit 44, and an operating section 51.

The CPU 41 is connected to respective components through a not-shown data-bus or address-bus or control line, so as to control the overall operation of the processor 13 in a comprehensive manner. ROM 52 stores various programs and data for controlling the operation of the processor 13, such as OS, applications and graphic data. The CPU 41 reads out the program and data as needed from the ROM 52, develops the read program and data on a work memory or RAM 53 as to execute the program sequentially. The CPU 41 also obtains variable information about each individual examination, including the data of examination, the subject or patient to be examined, and the doctor or operator in charge of the endoscopic procedure, from the operating section 51 or through a network such as a local area network (LAN), and stores the obtained information in the RAM 53.

The DSP 42 processes the image signal output from the CCD 21, for color-separation, color-interpolation, gain correction, white-balance control, gamma correction, and other various signal processing to produce image data. The image data from the DSP 42 is written in a work memory of the DIP 43. The DSP 42 also produces data for automatic light control (ALC) that is necessary for automatically controlling the volume of the illumination light. The ALC data may for example be an average luminance value of all pixels of the produced image.

The DIP 43 is processing the image data for electronic zooming, color enhancement, edge enhancement, and other various image processing. The image data processed by the DIP 43 is stored temporarily in the VRAM 28, and is fed to the display control circuit 44 to display the endoscopic image visualizing the interior of the subject body.

The DIP 43 also has a halation detector 55 for detecting whether halation occurs in the captured endoscopic image. The halation detector 55 compares pixel levels, i.e. luminance values, of the respective pixels of the captured image with a predetermined threshold value, to count the number of those pixels having larger values than the threshold value. When the number of those pixels having larger values than the threshold value is above a predetermined number, the halation detector 55 judges that halation occurs in the image. Thus, the halation detector 55 checks every captured image as to whether it has halation or not.

Hereinafter, those images which are judged to have halation will be referred to as images with halation, and those which are not judged to have halation will be referred to as images without halation.

The result of judgment by the halation detector 55 is fed to the CPU 41. Based on the result of judgment by the halation detector 55, the CPU 41 determines those images to be displayed on the monitor 22 among the images stored in the VRAM 28. Then the CPU 41 sends an image selection signal to the display control circuit 44, instructing that image data of only those images determined to be displayed on the monitor 22 should be read out from the VRAM 28.

The VRAM 28 is a temporary memory for memorizing the image data from the DIP 43 temporarily. For example, the VRAM 28 can store five image frames at a time. When the VRAM 28 already contains a maximum number of images for its memory capacity, e.g. five frames, the DIP 43 deletes the first-stored image and writes a new image in the VRAM 28, for example by overwriting the new image on the first-stored image. Accordingly, the VRAM 28 has a capacity to memorize a number of captured images, including the latest image. From among the images memorized in the VRAM 28, the display control circuit 44 selects one image to display it on the monitor 22 according to the image selection signal from the CPU 41.

Especially while photodynamic therapy (PDT) is being carried out, the latest image without halation will be picked up and displayed from among the images memorized in the VRAM 28. The display control circuit 44 receives the graphic data and other data from the CPU 41. The graphic data, which are stored in the ROM 52 and the RAM 53, include a display mask for masking out other areas than an effective area containing the subject, character information for displaying the date of examination and information about the subject and the operator, and graphic user interfaces (GUI). The display control circuit 44 superimposes the graphic data on the image and then converts it to a video signal, e.g. component signal or composite signal, compatible to the display format of the monitor 22. Thus, the selected image is displayed with the graphic data on the monitor 22.

The operating section 51 may consist of well-known input devices such as an operation panel mounted on a housing of the processor 13, a mouse, and a keyboard. The operating section 51 also includes a treadle switch for starting and stopping projection of a therapeutic light for the PDT. The CPU 41 operates the respective components of the electronic endoscope system 11 according to operational signals from the operating section 51 as well as those from the handling section 17 of the electronic endoscope 12.

In addition to the above mentioned components, the processor 13 includes a compressor circuit for compressing the image data to a predetermined compressed format, e.g. JPEG format, a media interface for recording the compressed image in a removable media, and a network interface for controlling data transmission over the LAN or other networks, etc. These components are connected to the CPU 41 via a data bus or the like.

The light source apparatus 14 includes an illumination light unit 61 and a therapeutic light unit 62. The illumination light unit 61 is for projecting illumination light to the inside of the subject body during the imaging. The illumination light unit 61 has a light source 63, a stop mechanism 64, and a wavelength filter 65.

The light source 63 emits broadband light having a broad wavelength range from red to blue, e.g. from around 400 nm to 800 nm. The broadband light may also be called white light and will be referred to as the ordinary light. The light source 63 may for example be a xenon lamp or a halogen lamp that emits the ordinary light at a constant intensity. The stop mechanism 64 adjusts the volume of the ordinary light from the light source 63. The wavelength filter 65 may limit the ordinary light to a narrow wavelength range that excites a photosensitive substance or photosensitizer to emit fluorescent light, the photosensitizer being previously injected in the subject body for the sake of PDD and PDT. The narrow band light limited the narrow wavelength range will be called the exciting light. The illumination light from the light source 63, the ordinary light or the exciting light, will be converged by a condenser lens 67, to be introduced into an inlet of the light guide 32.

The light guide 32 may for example be made of multiple quartz optical fibers bundled by a winding tape. The illumination light conducted through the light guide 32 is diffused through the projection lens 33 at an outlet of the light guide 32 when projected into the subject body.

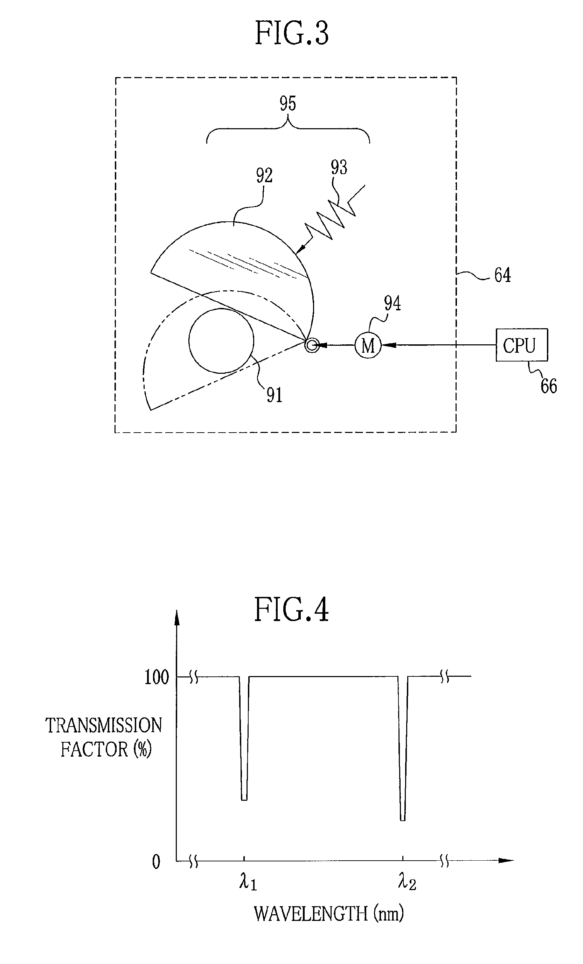

The stop mechanism 64 includes a stop aperture 91 (see Fig.3) and a stop blade 92 (see Fig.3) for opening and closing the stop aperture 91. As set forth in detail later, the stop mechanism 64 adjusts the volume of the illumination light automatically under the control of the CPU 66.

The wavelength filter 65 is a filter for limiting the ordinary light from the light source 63 to the exciting light. For example, the wavelength filter 65 has a semi-circular shape and may be driven by a motor to swing across between the light source 63 and the condenser lens 67. The wavelength filter 65 is provided with a sensor for detecting the rotational position thereof. While the wavelength filter 65 is moving across between the light source 63 and the condenser lens 67, the exciting light is projected as the illumination light into the subject body. So long as the wavelength filter 65 does not intersect the light path between the light source 63 and the condenser lens 67, the ordinary light is projected as the illumination light into the subject body. The objective lens system 31 controls the wavelength filter 65 to switch over between the ordinary light and the exciting light in accordance with instructions entered through the handling section 17 to change the observation mode.

The therapeutic light unit 62 is a unit for projecting the therapeutic light toward tumor cells which have accumulated the photosensitive substance for the PDT.

The therapeutic light unit 62 has a light source 71 that radiates red rays of 630 nm to 680 nm. Since the red ray from the light source 71 have a therapeutic effect to the tumor, the red ray from the light source 71 may be called the therapeutic light. The light source 71 may for example be a laser diode (LD) that may radiate a pulsed laser beam at a high intensity in comparison with the ordinary light or the exciting light for the PDD. The volume of the therapeutic light from the light source 71 may be adjusted by a stop mechanism 72. Thereafter, the therapeutic light is converged by a condenser lens 77, to be introduced into a treatment probe or catheter 73.

The stop mechanism 72 may have the same structure as the stop mechanism 64 of the illumination light unit 61, opening and closing a stop aperture 91 with a stop blade 92, thereby to adjust the volume of the therapeutic light before it is introduced into the treatment probe 73. The operation of the stop mechanism 72 is automatically controlled by the CPU 66.

The treatment probe 73 is a treatment tool for use in the PDT. The treatment probe 73 conducts the therapeutic light from the light source apparatus 14 to project the therapeutic light from a distal end toward the tumor cells that have accumulated the photosensitizer. A proximate end of the treatment probe 73 is coupled to the light source apparatus 14 such that the therapeutic light from the therapeutic light unit 62 is introduced into the treatment probe 73. The distal end of the treatment probe 73 is inserted into the probing portion 16 of the electronic endoscope 12 from the tool inlet 74, and is conducted through the tool channel 75 in the probing portion 16, to protrude out of the toll outlet 76 at the tip 20 of the probing portion 16. The tool outlet 76 is an open end of the tool channel 75. Pushing or pulling the treatment probe 73 at the tool inlet 74 may appropriately adjust the protruded amount of the treatment probe 73 from the tool outlet 76.

The CPU 66 communicates with the CPU 41 of the processor 13 to control the operations of the stop mechanisms 64 and 72 and the wavelength filter 65, to make automatic light control (ALC) of the volume of the illumination light according to the operation modes. The CPU 66 executes the ALC based on control data for the ALC that is produced by the DSP 42.

A hood 81 is attached to the distal end of the probing portion 16. The hood 81 has an imaging window 82, a lighting window 83, and an opening 84 in corresponding positions to the imaging window 23, the illumination window 24, and the tool outlet 76 of the tip 20, respectively.

A light-reduction filter 85 is provided in the imaging window 82. The light-reduction filter 85 is a notch filter for reducing the intensity of both the exciting light for the PDD and the therapeutic light for the PDT. That is, the light-reduction filter 85 lets substantially 100% of light components of other wavelength bands than the exciting light and the therapeutic light pass through it. As a result, even while the exciting light reflected from the interior of the subject body is entering through the imaging window 82 during the PDD in addition to the fluorescent light generated from the photosensitizer, the exciting light is dimmed through the light-reduction filter 85. Thus, an image of the interior of the subject body will be captured based on the fluorescent light whose intensity is smaller than the original intensity of the exciting light.

During the PDT, the therapeutic light is projected at a high intensity for the sake of shorter treatment time. The light-reduction filter 85 also reduces the volume of the therapeutic light as it is reflected from the interior of the subject body and falls on the CCD 21.

As the ordinary light is a broadband light covering the wavelength ranges of the exciting light and the therapeutic light, the light-reduction filter 85 will reduce the light components of the same wavelength ranges as the exciting light and the therapeutic light during the imaging under the illumination of the ordinary light (the ordinary light imaging). However, because almost all of other light components will penetrate the light-reduction filter 85, an image captured by the ordinary light imaging will not be substantially affected by the presence of the light-reduction filter 85.

The lightening window 83 has a transparent material, e.g. a glass plate, fitted therein, which lets at least the ordinary light and the exciting light pass through it. Therefore, the ordinary light and the exciting light can be projected into the subject body in the same way regardless of whether the hood 81 is attached to the tip 20 or not.

The opening 84 exposes the entire tool outlet 76 when the hood 81 is attached to the tip 20, so that the treatment probe 73 or other treatment tool may produce out of the tip 20 even while the hood 81 is attached to the tip 20.

As shown in Fig. 3, the stop mechanism 64 of the illumination light unit 61 includes a stop adjusting mechanism 95 consisting of the stop blade 92, a spring 93 urging the stop blade 92 toward a position closing the stop aperture 91, and a motor 94. The motor 94 applies torque to the stop blade 92 to swing in a direction to open the stop aperture 91 against the urging force of the spring 93, i.e. in a clockwise direction in the drawings. The stop blade 92 stops at a position where the torque balances with the urging force of the spring 93. As the torque increases, the force against the urging force of the spring 93 will crease, opening the stop aperture 91 wider. As the torque decreases, the force against the urging force of the spring 93 will decrease, reducing the size of the stop aperture 91.

The torque of the motor 94 is controlled according to the ALC (automatic light control) data from the DSP 42. The CPU 66 of the light source apparatus 14 controls the stop adjusting mechanism 95. According to the ALC data, the CPU 66 calculates a pulse width modulation (PWM) value of drive pulses which are applied from a not-shown motor driver to the motor 94. The PWM value decides the duty ratio of the drive pulses, i.e. the ratio of pulse width to pulse interval. The torque of the motor 94 will increase as the PWM value increases. When the ALC data requests an increase in the stop aperture size, the CPU 66 boosts the PWM value in accordance with the requested increase. When the ALC data requests a decrease in the stop aperture size, the CPU 66 lowers the PWM value accordingly.

The stop mechanism 72 of the therapeutic light unit 62 has the same structure as the stop mechanism 64 of the illumination light unit 61, and is controlled by the CPU 66. However, the CPU 66 controls the stop mechanism 72 to close the stop aperture 91 entirely to interrupt projection of the therapeutic light for one frame period in response to an interruption signal from the CPU 41 of the processor 13. During other periods, the CPU 66 controls the stop mechanism 72 to open up the stop aperture 91 of the stop mechanism 72.

The CPU 41 outputs the interruption signal to the CPU 66 as soon as the successive four of the latest five images stored in the VRAM 28 are judged to have halation. The CPU 41 counts the number of judgment signals applied from the halation detector 55 to indicate the occurrence of halation in a stored image, and the CPU 41 outputs the interruption signal when it has counted four judgment signals successively.

Accordingly, if four images with halation have been successively stored in the VRAM 28, projection of the therapeutic light is interrupted during the next frame period, so that an image without halation may be captured in the next frame period. Thus, even while the PDT is being executed, at least one of five images stored in the VRAM 28 is free from halation.

As shown in Fig.4, the light-reduction filter 85 has lower transmission factors at around the wavelength λ1 of the exciting light and around the wavelength λ2 of the therapeutic light, but transmits approximately 100% of other wavelength rays. The light-reduction filter 85 is fabricated by overlaying a first notch filter with a second notch filter, the first notch filter having the low transmission factor at around the wavelength λ1 of the exciting light, and the second notch filter having the low transmission factor at around the wavelength λ2 of the therapeutic light. The transmission factor of the exciting light at the wavelength λ1 is determined according to the intensity of the exciting light for the PDD and the fluorescent light, whereas the transmission factor of the therapeutic light at the wavelength λ2 is determined by the intensity of the therapeutic light for the PDT. Note that the light-reduction filter 85 may be fabricated in an appropriate manner, for example by forming the first and second notch filters as optical thin film layers, insofar as it can dim the exciting light and the therapeutic light.

The operation of the electronic endoscope system 11 configured as above will be described. Prior to making the PDD and PDT with the electronic endoscope system 11, the operator should dose the patient with the photosensitizer. When a certain time has elapsed after the dose of the photosensitizer enough for the photosensitizer to be sufficiently accumulated in tumor cells, the operator starts the inspection and the treatment by actuating the processor 13 and the light source apparatus 14 after connecting them to the electronic endoscope 12. The operator also connects the proximate end of the treatment probe 73 to the light source apparatus 14 and inserts the distal end of the treatment probe 73 through the tool inlet 74 into the electronic endoscope 12. The operator also inputs necessary information about the patient through the operating section 51. Then the operator attaches the hood 81 to the tip 20 of the probing portion 16 and introduces the probing portion 16 into the patient while illuminating the interior or body cavity of the patient with the ordinary light to observe the interior of the patient.

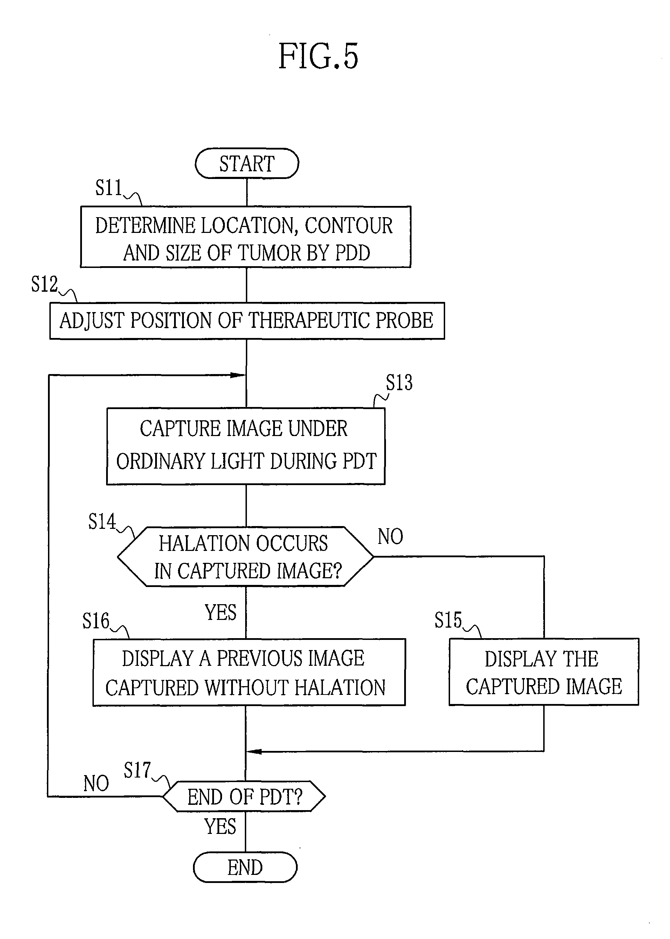

When the tip 20 comes close to the aimed tumor site, the operator operates the handling section 17 to switch the system 11 from the ordinary observation mode to the PDD mode. When the system 11 is switched to the PDD mode, the CPU 41 turns the wavelength filter 65 to the position for projecting the exciting light into the patient body. Then the monitor 22 displays an image captured under the illumination of the exciting light, hereinafter referred to as PDD image. Since the photosensitizer emits the fluorescent light as it is exposed to the exciting light, the PDD image visualizes the tumor cells as accumulating the photosensitizer therein. Thus the operator can perceive the location, size, geometry and other features of the tumor from the PDD image (step S11 in Fig.5).

Thereafter, the operator may change the relative positions of the tip 20 and the tool outlets 76 and 84 to the tumor site by pushing or pulling or rotating the probing portion 16. Then the operator protrudes the treatment probe 73 out of the tool outlets 76 and 84, and then adjusts the distance and orientation of the treatment probe 73 to the tumor site such that the projection range of the therapeutic light covers the entire tumor cells (step S12).

When the positioning of the treatment probe 73 is complete, the operator steps on the treadle switch of the operating section 51 in order to project the therapeutic light from the distal end of the treatment probe 73 to start the photodynamic treatment (PDT) of the tumor. Stepping on the treadle switch of the operating section 51 feeds an irradiation start signal to the CPU 41 of the processor 13, causing the light source 71 of the therapeutic light unit 62 to emit the therapeutic light. The CPU 41 also controls the stop mechanism 72 to open up the stop aperture 91 to project the therapeutic light at the maximum volume. Simultaneously, the CPU 66 of the light source apparatus 14 turns the wavelength filter 65 to a position for letting the ordinary light from the illumination light unit 61 into the light guide 32. Consequently, while the PDT is being conducted by projecting the therapeutic light toward the tumor, the ordinary light is projected into the patient body to conduct the ordinary light imaging under the illumination of the ordinary light (step S13).

Because the therapeutic light is projected at a high intensity, the reflected therapeutic light also has such intensity even after being reduced through the light-reduction filter 85 that it may cause halation in a image captured during the PDT (hereinafter referred to as the PDT image). In order to detect whether the PDT image suffers halation or not, the halation detector 55 compares luminance values of the respective pixels of the PDT image with the predetermined threshold value each time the PDT image is supplied from the DSP 42. The halation detector 55 counts the number of those pixels having larger values than the threshold value within the PDT image, to judge that the PDT image suffers halation if the number of pixels having larger values than the threshold value is above a predetermined value. The result of judgment is fed to the CPU 41 (step S14).

If the latest PDT image is an image without halation, the CPU 41 outputs an image selection signal to the display control circuit 44 to select the latest PDT image as an image to be displayed on the monitor 22. Then the display control circuit 44 displays the latest PTD image on the monitor 22 (step S15).

On the other hand, if the latest PDT image is an image with halation, the CPU 41 temporarily stores the latest PDT image in the VRAM 28 but supplies the display control circuit 44 with an image selection signal that instructs to select the latest one of those PDT images which has been captured without halation among the five images stored in the VRAM 28. According to the image selection signal, the display control circuit 44 displays the latest image having no halation on the monitor 22 (step S16).

Thus, the electronic endoscope system 11 continually displays the PDT images without halation on the monitor 22 during the PDT.

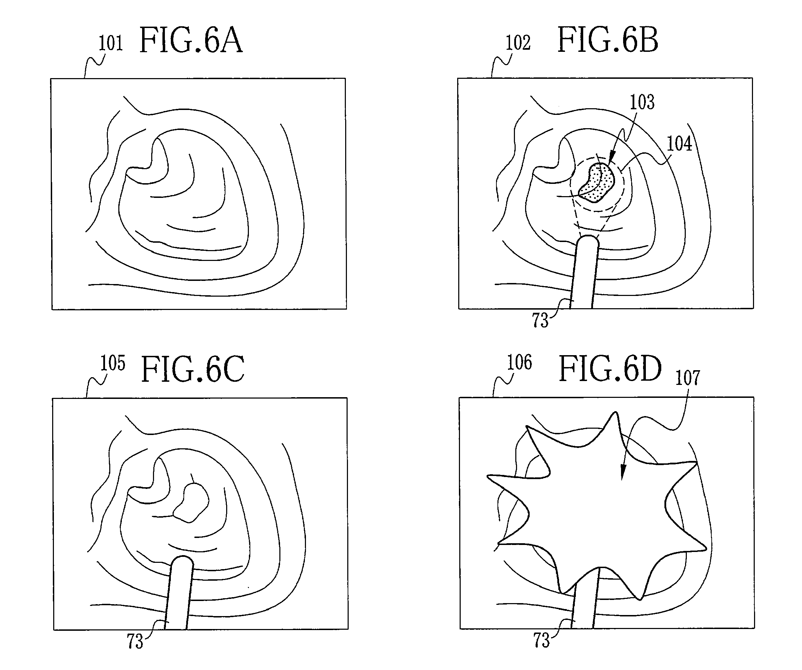

Referring to Fig.6A, an example of an ordinary light image 101 captured under the illumination of the ordinary light alone is illustrated. The ordinary light image 101 clearly visualizes the interior of the subject body. However, even if there is a tumor in the subject body and the photosensitizer previously accumulated in the tumor cells emit fluorescence responding to the exciting light component included in the ordinary light, the intensity of the fluorescent light emitted from the photosensitizer is so low in comparison with the total intensity of the ordinary light that it is difficult to discriminate the tumor cells from other tissues or organs in the ordinary light image 101.

In contrast, as shown in Fig.6B, a PDD image 102, which is displayed on the monitor 22 during the PDD (steps S11 and S12), clearly shows the tumor cells 103, because the PDD image 102 is formed by the fluorescent light emitted from tumor cells 103 and the reflected exciting light whose intensity is reduced through the light-reduction filter 85 to or below the level of the fluorescent light. When the treatment probe 73 is protruded out of the tip 20 of the probing portion 16, the distal end of the treatment probe 73 will be visible in the PDD image 102. Thus, the operator can adjust the position and distance of the distal end of the treatment probe 73 relative to the tumor cells 103 so as to locate the tumor cells 103 within a projection field 104 of the therapeutic light.

Two examples of the PDT images are shown in Figs. 6C and 6D, which are captured under the illumination of the ordinary light during the PDT while the therapeutic light is being projected in the steps S13 to S16. As shown in Fig.6C, depending upon the situation, the PDT image 105 can be captured without any halation like the ordinary image 101. However, as shown by the PDT image 106 in Fig. 6D, halation 107 can occur in and around the projection field 104 of the therapeutic light in many of the PDT images. The halation 107 will bleach out the main portion of the PDT image around the tumor cells, making the tumor opaque or invisible. In that case, the operator cannot observe the features of the tumor or confirm the therapeutic effect of the PDT.

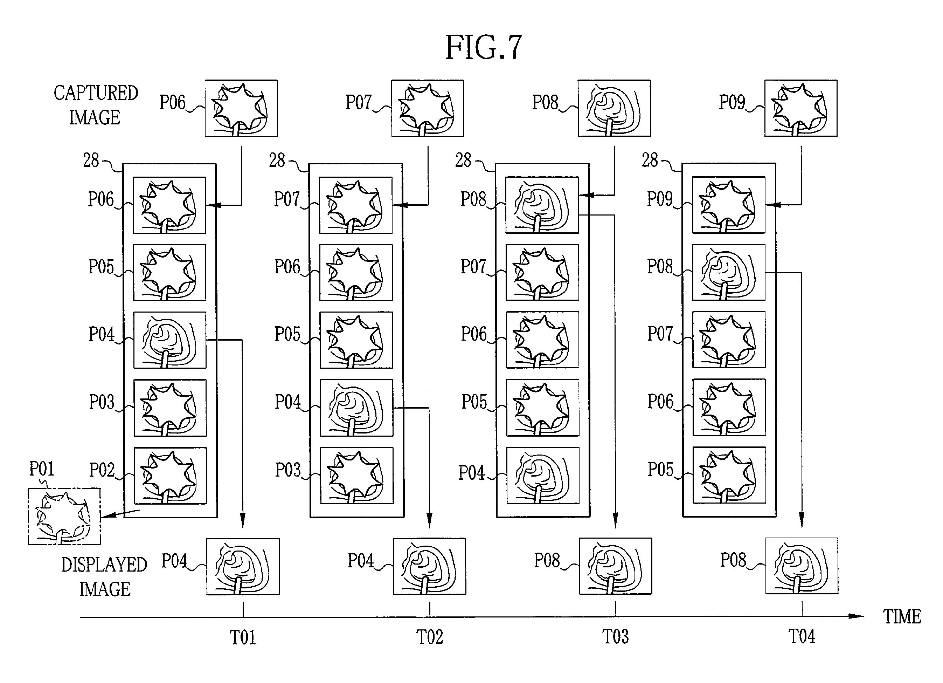

Referring to Fig.7, the relation between the PDT images captured in the steps S13 to S16 and the images displayed on the monitor 22 during the PDT is illustrated for example.

This example assumes that a PDT image P06 with halation is captured in a frame at a time T01 and the VRAM 28 stores five PDT images P01 to P05 which have been sequentially captured just before the PDT image P06, wherein the PDT images P01 to P03 and P05 have halation, and the PDT image P04 does not have halation.

When the PDT image P06 is captured in the frame at the time T01, the DIP 43 activates the halation detector 55 to determine whether there is halation in the PDT image P06. The DIP 43 also erases the oldest PDT image P01 from the VRAM 28 among the stored PDT images P01 to P05, and writes the latest PDT image P06 in the VRAM 28. Accordingly, the VRAM 28 stores the PDT images P02 to P06 upon the PDT image P06 being captured.

In this case, the CPU 41 supplies the display control circuit 44 with an image selection signal instructing to select the PDT image P04 from among the stored PDT images P02 to P06 because the CPU 41 determines by the judgments from the halation detector 55 that only the PDT image P04 is free from halation. Consequently, the monitor 22 displays the PDT image P04 without halation at the time T01 even though halation occurs in the PDT image P06 captured at the time T01.

When another PDT image P07 is captured in the next frame at a time T02, the oldest PDT image P02 of the stored images P02 to P06 is erased from the VRAM 28, and the latest PDT image P07 is written in the VRAM 28. If the latest PDT image P07 suffers halation, the CPU 41 instructs the display control circuit 44 to select the PDT image P04 as the image without halation from among the stored PDT images P03 to P07. Therefore, the PDT image P04 is displayed on the monitor 22 at the time T02 continually from the time T01.

When a new PDT image P08 is captured in the next frame at a time T03, the PDT image P08 is written in the VRAM 28 in place of the oldest PDT image P03. If the latest PDT image P08 is without halation, the VRAM 28 stores two PDT images having no halation. Then the CPU 41 instructs the display control circuit 44 to select the latest one of the images without halation from among the images stored in the VRAM 28. Accordingly, the display control circuit 44 selects the PDT image P08 to be displayed on the monitor 22. Thus, the latest PDT image P08 is displayed at the time T03.

If a PDT image P09 captured in the next frame at a time T04 is an image with halation, the latest PDT image P08 among the images without halation is read out from the VRAM 28, to be displayed on the monitor 22.

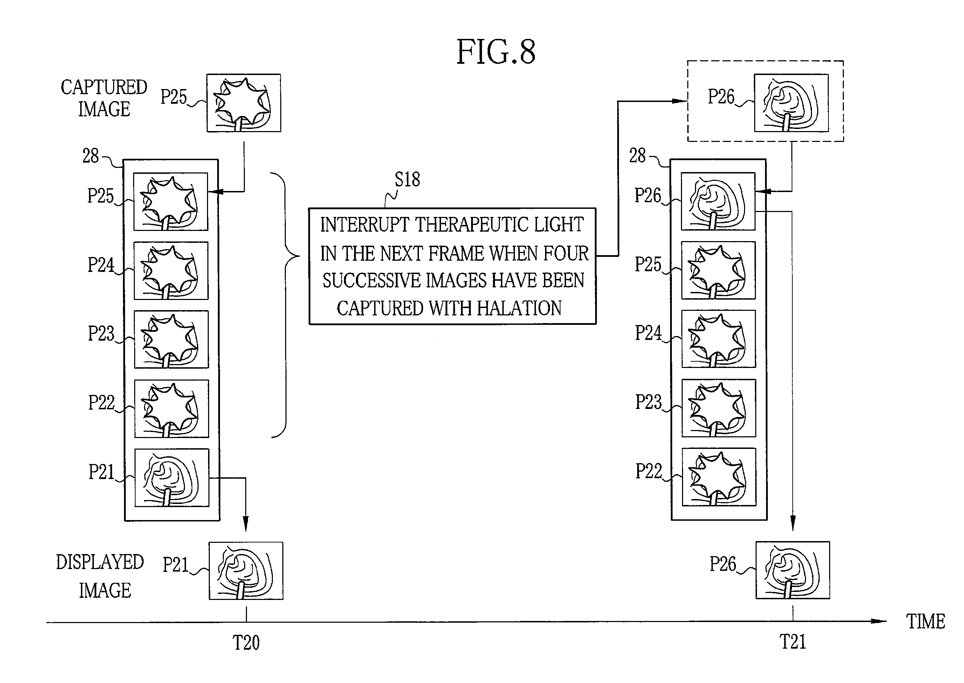

As shown in Fig.8, when a PDT image P25 captured at a time T20 is with halation and the latest four PDT images P22 to P25 captured successively by the time T20 are all with halation, while only the oldest PDT image P21 among the stored ones is without halation, the PDT image P21 without halation is selected to be displayed on the monitor 22 at the time T20.

However, if halation occurs in the next PDT image that will be captured in the next frame, the PDT image P21 without halation will be erased from the VRAM 28 and the VRAM 28 will not contain any images without halation. Then the monitor 22 cannot but display the image with halation.

In order to avoid this inconvenience, the CPU 41 counts the number of judgment signals from the halation detector 55 indicating the occurrence of halation in the stored images. When it has counted four judgment signals in continuous succession, i.e. when it is determined that successive four images have been captured with halation, the CPU 41 outputs the interruption signal to the CPU 66 to interrupt projection of the therapeutic light during the next frame.

Therefore, in the next frame at a time T21 next to the time T20 in the example of Fig.8, an image P26 is captured under the illumination of the ordinary light alone. Without the therapeutic light, no halation will occur in the image P26. As the image P26 without halation is written in the VRAM 28, the image P26 is displayed on the monitor 22.

As described so far, the electronic endoscope system 11 interrupts projection of the therapeutic light forcibly to capture an image without halation in the next frame, as soon as four images have been captured with halation in continuous succession. Thus, the electronic endoscope system 11 ensures that at least one of five images stored in the VRAM 28 is without halation, so that the monitor 22 can always display endoscopic images without halation.

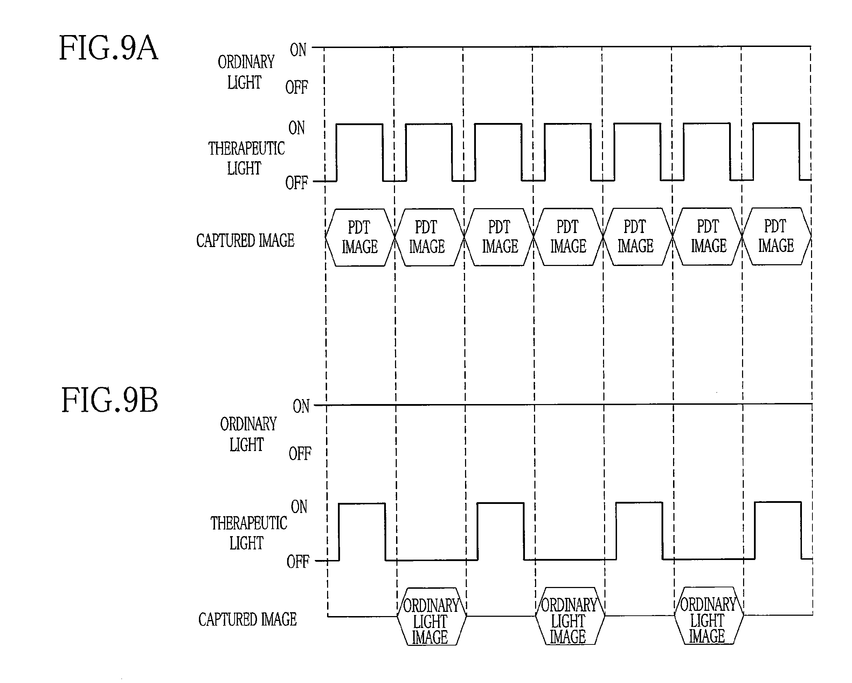

As shown in Fig.9A, the electronic endoscope system 11 illuminates the interior of the subject body continually with the ordinary light to capture and display endoscopic images during the PDT, while irradiating the tumor cells with the therapeutic light in every frame. Even when a PDT image has halation, the latest one of those preceding images having no halation is selected from among five images stored in the VRAM 28, and the selected image without halation is displayed on the monitor 22. Therefore, the electronic endoscope system 11 keeps displaying endoscopic images without halation even in the PDT mode just like in the ordinary observation mode.

In a comparative case, as shown in Fig.9B, the therapeutic light is projected in every other frame, and images are captured only in those frames where the therapeutic light is not projected. Thereby, the captured images will be free from halation, and every captured image may be displayed sequentially on the monitor 22.

Indeed the comparative case as well as the electronic endoscope system 11 prevent displaying images with halation, the comparative case can project the therapeutic light only in every other frame while the electronic endoscope system 11 can project the therapeutic light in every frame at the maximum. That is, the amount of the therapeutic light the electronic endoscope system 11 can project per hour would be up to twice the comparative case. Namely, the electronic endoscope system 11 may optimally reduce the requisite time for the PDT to half the comparative case.

As described above, if successive four images have halation during the PDT, the electronic endoscope system 11 interrupts projection of the therapeutic light in the next frame, to capture an image without halation at least once in five successive frames, thereby to continue displaying images without halation on the monitor 22. Even where the therapeutic light is interrupted once in five frames, the electronic endoscope system 11 can project the therapeutic light in an amount her hour that is 8/5 times the comparative case and hence the requisite time for the PDT will be reduced to 5/8 of the comparative case. The PDT usually takes a relatively long time, i.e. around 10 minutes, though it depends on the tumor size and the power of the therapeutic light. Therefore it is desirable to shorten the treatment time in order to reduce the load on the patient.

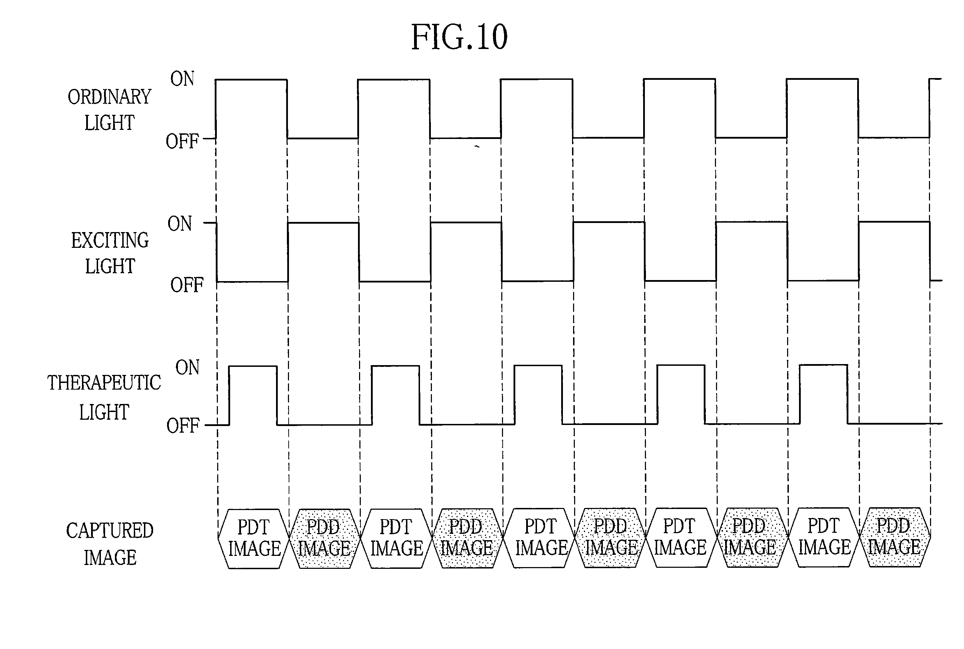

In the above first embodiment, PDT images are captured during the PDT. Without any halation, the PDT images look substantially similar to the ordinary light images, and they do not show the tumor cells conspicuously as fluorescent parts. In order to make the tumor cells distinguishable on the monitor 22 during the PDT, it is preferable to project the exciting light to display the PDD image during the PDT. However, because the wavelength of the fluorescent light from the photosensitizer, which has been accumulated in the tumor cells and emits the fluorescent light when excited by the exciting light, is near to the wavelength of the therapeutic light, and the intensity of the therapeutic light is extremely larger than that of the fluorescent light, it is impossible to make the PDT and the PDD at the same time. In order to capture the PDD image in the PDT mode, it is possible to turn off the ordinary light and the therapeutic light in every other frame during the PDT and project the exciting light alternately with the ordinary light and the therapeutic light, as shown in Fig. 10. Thus the PDT images and the PDD images may be captured alternately with each other. This configuration will save the treatment time in comparison with a case where the ordinary light imaging, the PDT image capturing and the PDD image capturing are executed one after another in individual frames. The PDT image and the PDD image may preferably be displayed in parallel on the monitor 22 when they are captured alternately in the PDT mode.

As described above, the VRAM 28 is configured to store a certain number of PDT images, i.e. five frames in the first embodiment, in order to prevent displaying an image with halation on the monitor 22. In the case where the PDD images are captured alternately with the PDT images, the VRAM 28 should have a capacity to store at least a frame of the PDD image in addition to the PDT images. It is alternatively possible to provide a subsidiary frame memory for storing the PDD image.

It may be preferable to increase the intensity of the therapeutic light, for example by boosting up the power of the light source 71, in any of the four frames succeeding to the frame in which the therapeutic light is forcibly interrupted to capture an image without halation. Once the VRAM 28 has stored at least one image without halation, halation is acceptable in the succeeding four image frames. Increased intensity of the therapeutic light will make up for the reduction of irradiation time by the interruption of the therapeutic light and is useful for keeping the treatment time short.

In the above first embodiment, the monitor 22 always displays images without halation. In that case, it is difficult for the operator to perceive the therapeutic light itself and check whether the therapeutic light is being projected exactly onto the tumor site. The effect of the therapeutic light can only be evaluated from changes in the tumor cells shown by the PDT images. Therefore it is desirable to display information about the therapeutic light, such as the location irradiated with the therapeutic light.

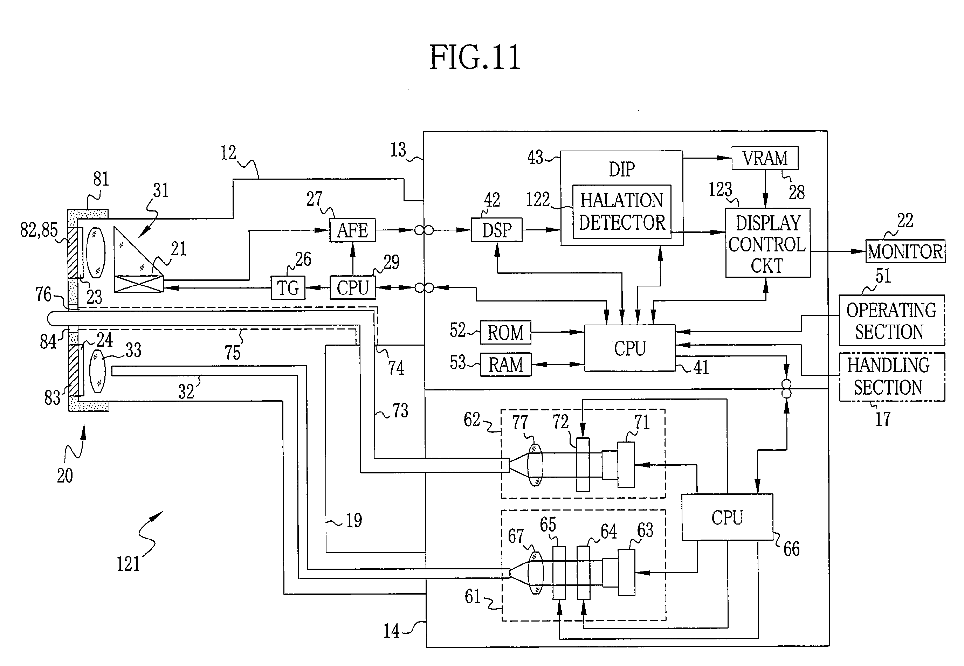

According to a second embodiment shown in Fig.11, an electronic endoscope system 121 has a halation detector 122 that detects halation in captured endoscopic images in the same way as the halation detector 55 of the first embodiment, but also extracts data about features of the detected halation, including the contour of the halation, i . e . an edge of a zone that is detected as a group of pixels having larger luminance values than a certain threshold value. The feature data of the halation is fed from the halation detector 122 to a display control circuit 123. If the halation detector 122 does not detect any halation in the latest captured image, the halation detector 122 feeds the latest feature data extracted from previous endoscopic images to the display control circuit 123. The display control circuit 123 selects the latest image without halation from among PDT images stored in a VRAM 28 based on an image selection signal from a CPU 41, and superimposes the feature data onto the selected image, to display the image without halation and the superimposed feature data on a monitor 22.

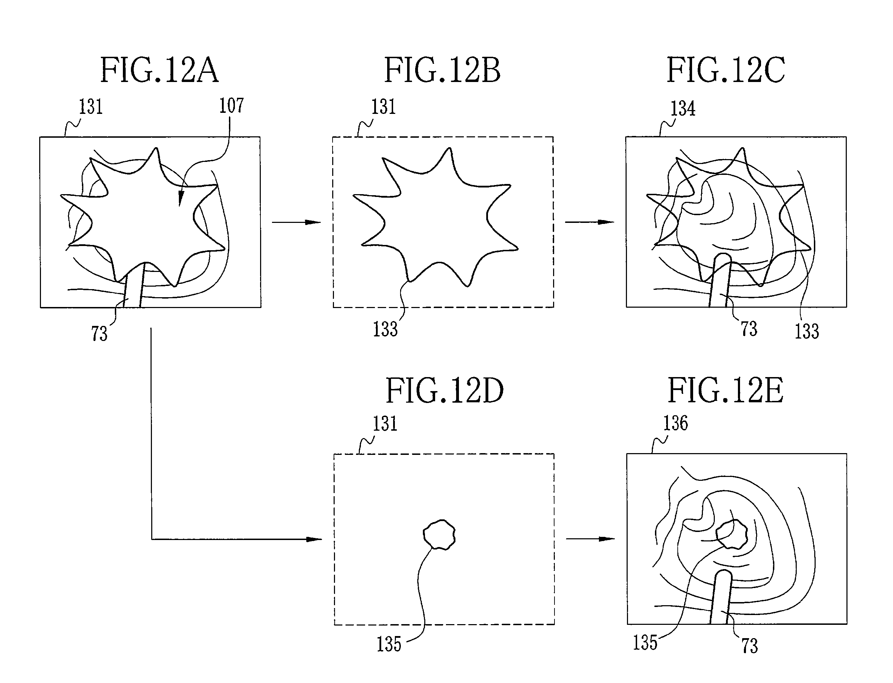

For example, as shown in Figs. 12A and 12B, when a PDT image 131 is captured with halation 107, the halation detector 122 detects those pixels having larger values than the predetermined threshold value to identify them as halation or an overexposed area of the image and extract a contour 133 of the halation 107, i.e. an edge of the extremely bright area, as the feature data of the halation 107. The display control circuit 123 selects the latest image without halation from the VRAM 28, and displays a composite image 134 on the monitor 22, as shown in Fig. 12C, wherein the contour 133 of the halation 107 is superimposed on the image without halation.

Alternatively, as shown in Fig. 12D, an edge or contour of a projection spot 135 of the therapeutic light may be extracted as feature data from the PDT image 131. The contour of the projection spot 135 may be extracted for example by comparing respective pixel levels with a higher threshold value than the threshold value for the halation detection. Extraction of the contour of the projection spot 135 may be done by the display control circuit 123 simultaneously with the detection of the halation 107.

Displaying a composite image 136 having the contour of the projection spot 135 superimposed on the image without halation, as shown in Fig. 12E, allows the operator to perceive the location and range of irradiation more clearly than in the case where the contour 133 of the halation 107 is superimposed on the displayed image.

In either case, however, the operator can obtain information about the therapeutic light and confirm whether the therapeutic light is projected from a treatment probe 73, whether the therapeutic light exactly irradiates the tumor site, or whether the irradiation range covers the entire tumor cells.

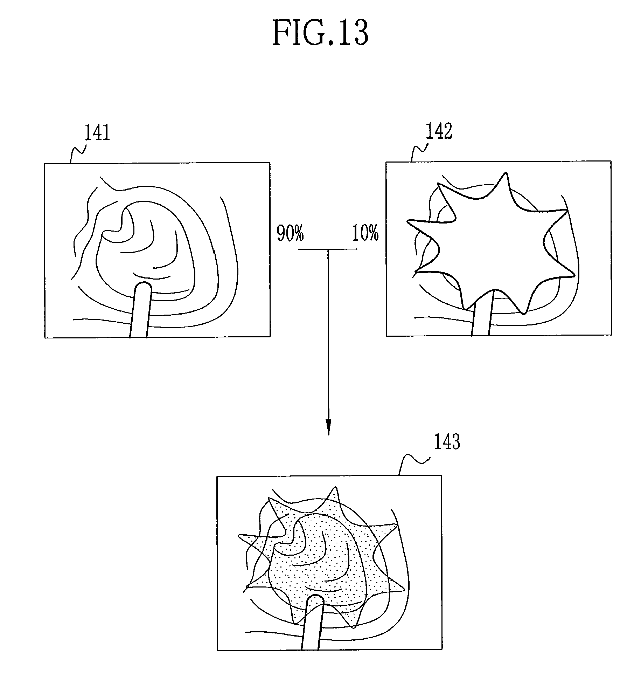

In an alternative, as shown in Fig. 13, a previous PDT image 141 having no halation may be combined with the latest PDT image 142 that has halation, to produce and display a mixed image 143. For example, the mixed image 143 is produced by adding 10% of the values of the respective pixels of the latest PDT image 142 to 90% of the values of the corresponding pixels of the previous PDT image 141. Thus, the mixed image 143 may show not only the interior of the subject body and the tumor but also information relating to the therapeutic light. The mixed image 143 may be produced in a display control circuit 123, or a specific circuit for combining the PDT images 141 and 142 in this way may be provided for example in a DIP 43. It is also possible to produce a mixed image by combining a fragment of the latest PDT image 142, including an area irradiated with the therapeutic light, with the previous PDT image 141 that the display control circuit 123 may read out from the VRAM 28.

In a case where the therapeutic light for the PDT is projected alternately with the exciting light for the PDD, like in the embodiment shown in Fig.10, it is possible to superimpose an image showing the contour of the halation or the projection spot onto the PDD image.

According to the above embodiments, the location and size of a tumor site may be determined from the PDD images, and the area irradiated with the therapeutic light may be identified by the superimposed image. However, since the efficiency of irradiation with the therapeutic light varies depending on the skill of the operator, it is desirable to visualize how the tumor cells are irradiated with the therapeutic light and thus enable the operator to judge objectively as to whether the therapeutic light efficiently acts on the tumor cells.

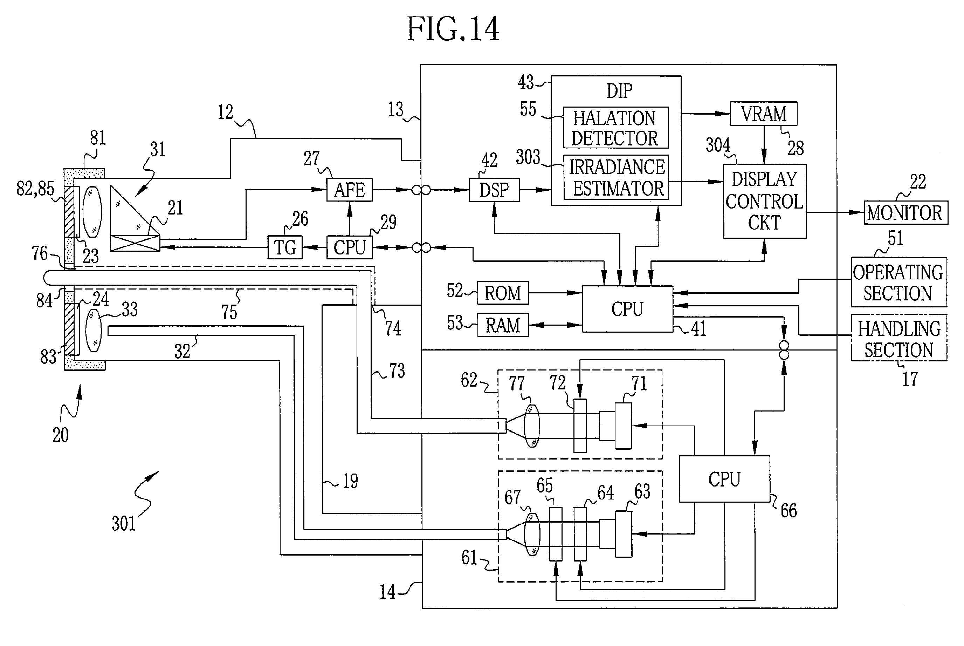

An embodiment shown in Fig.14 is intended to visualize the amount of therapeutic light projected onto the target site. Like the electronic endoscope system 11 of the first embodiment, an electronic endoscope system 301 of this embodiment displays a previous image without halation on a monitor 22 instead of the latest image captured during the PDT when the latest image has halation. Unlike the first embodiment, the electronic endoscope system 301 includes an irradiance estimator 303 and a display control circuit 304, and has a PDT preparation mode.

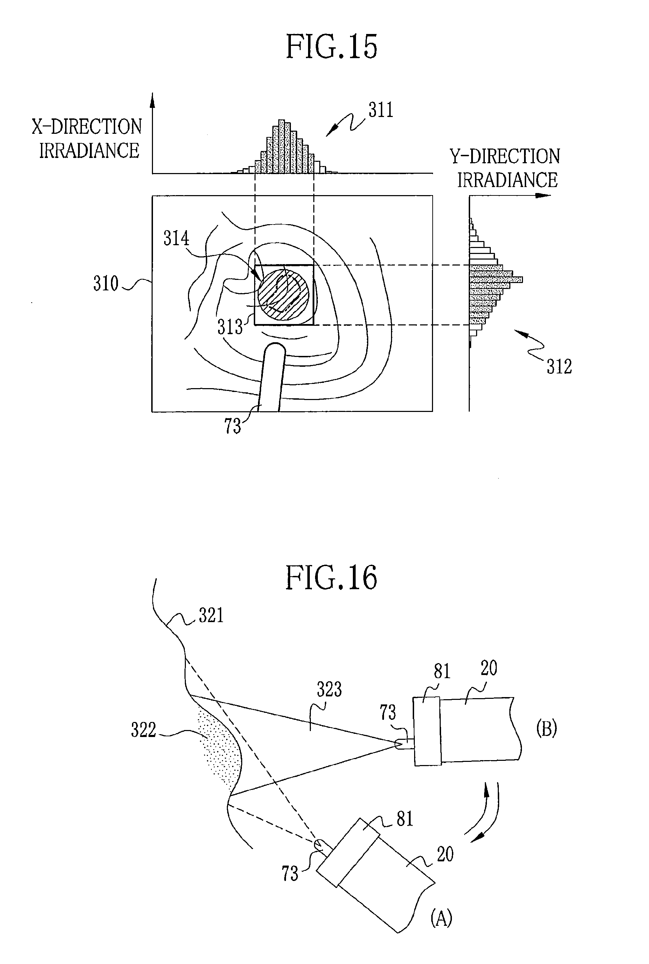

The PDT preparation mode is provided for adjusting the position and orientation of a tip 20 of an endoscope 12 and a treatment probe 73 relative to tumor cells before starting actual PDT (photodynamic treatment). When a command to start the PDT preparation mode is input through an operating section, a CPU 41 of a processing apparatus 13 applies a PDT preparation mode start signal to a CPU 66 of a light source apparatus 14. Then the CPU 66 controls a stop mechanism 72 to project the therapeutic light into the subject body at such a low intensity that no halation can occur. The low intensity therapeutic light is reflected from the interior of the subject body to form a projection spot in an image captured under the ordinary light in the PDT preparation mode, and this ordinary light image showing the projection spot is displayed on the monitor 22. The projection spot will change depending upon the current position and orientation of the tip 20 and the treatment probe 73. Moreover, a range of interest (ROI) is designated in the PDT preparation mode. For example, the ROI is a rectangular range designated to surround tumor cells, as designated by 313 in Fig. 15, wherein the tumor cells may have been located and dimensioned from PDD images captured during a PDD (photodynamic diagnosis) mode in advance to the PDT preparation mode.

The irradiance estimator 303 is actuated in the PDT preparation mode. The irradiance estimator 303 calculates coordinate values of a center pixel of the designated ROI to determine those red pixels which are on horizontal (X-direction) and vertical (Y-direction) lines intersecting at the center of the designated ROI. Then the irradiance estimator 303 extracts values of these red pixels in each image as captured under the ordinary light while the low intensity therapeutic light is being projected. The irradiance estimator 303 feeds data strings representative of the extracted values of the red pixels to the display control circuit 304. From the data strings received from the irradiance estimator 303, the display control circuit 304 produces histograms 311 and 312, as shown for example in Fig.15, and controls the monitor 22 to display the histograms 311 and 312 in association with the captured image 310.

The histogram 311 shows the distribution of estimated irradiances of the therapeutic light along the horizontal center line of the ROI 313. The histogram 312 shows the distribution of estimated irradiances of the therapeutic light along the vertical center line of the ROI 313. In the illustrated example, those bars of the histograms 311 and 312 which correspond to the pixels within the ROI 313, are highlighted using a different color or density. Moreover, a projection spot 314 of the therapeutic light is displayed in the image 310. Therefore, the operator can control the position and range of irradiation by adjusting the position and direction of the therapeutic light relative to the tumor cells while observing peak positions and peak values of the histograms 311 and 312 and the projection spot 314 in the image 310.

For example, as indicated by (A) in Fig.16, if the tip 20 of the probing portion 16 forms a small angle with respect to an inner wall 321 of the subject body, that is, if the low intensity therapeutic light 323 from the distal end of the treatment probe 73 falls on a tumor site 322 at a small incident angle, reflected part of the low intensity therapeutic light 323 will little fall on the CCD 21 even while the tumor site 322 is within the projection spot 314. As a result, irradiances indicated by the histograms 311 and 312 are generally low, which means the efficiency of irradiation by the therapeutic light is low. Besides that, because the amount of reflected light from a closer side of the tumor site 322 to the tip 20 will be greater than the amount of reflected light from a farther side of the tumor site 322 from the tip 20, the peak position of the histogram 311 or 312 will deviate from the center of the ROI (range of interest) 313. While observing these histograms 311 and 312, the operator may operate the angle knob of the handling section 17 to change the orientation of the tip 20 so as to be approximately perpendicular to the inner wall 321, as indicated by (B) in Fig.16. Then the amount of the reflected low intensity therapeutic light 323 from the tumor site 322 generally increases, and irradiances indicated by the histograms 311 and 312 get generally high. This means that the tumor site 322 is irradiated with the therapeutic light at a high efficiency. The irradiation range of the therapeutic light may be adjusted by pushing or pulling the probing portion 16 or the treatment probe 73 while checking the location of the projection spot 314. In practice, the inner wall 312 of the subject body will move, and the tip 20 and the treatment probe 73 should be adjusted so as to make the histograms 311 and 312 indicate as high irradiances as possible, and make the peak values as closer to the center of the ROI 313 as possible.

Visualizing the efficiency of irradiation of tumor cells with the therapeutic light as the histograms 311 and 312 in the PDT preparation mode allows objective judgment about the irradiation efficiency. Therefore the operator can adjust the position and orientation of the tip 20 and the treatment probe 73 so as efficiently to irradiate tumor cells with the therapeutic light. Consequently, the electronic endoscope system 301 improves the efficiency of the PDT and is effective to prevent the underexposure to the therapeutic light.

In the above embodiment, the operator designates the ROI 313 during the PDT preparation mode based on the location and size of the tumor site 322 estimated from the PDD images. In an alternative, feature points of the tumor site 322, representative of the location, size, contour and other features, may be extracted from the PDD images, so that the ROI 313 surrounding the tumor site 322 is automatically provided in the ordinary light image 310 on the basis of the extracted feature points. In that case, the extraction of the feature points of the tumor site from the PDD images may be done by a specific circuit, or the DIP 43 or the DSP 42 may function as a feature point extractor. Where the ROI 313 is automatically determined, the position of the ROI 313 should preferably follow the relative movement between the inner wall 321 of the subject body and the tip 20 or the treatment probe 73.

In the above embodiment, values of red pixels are extracted to produce the histograms 311 and 312 on the assumption that the therapeutic light is red. In another case where a photosensitizer reacting to another color ray is used, pixel levels of the corresponding color pixels may be extracted, or multiple color pixel levels may be summed up to produce irradiance histograms.

The embodiment shown in Figs. 14 to 16 has been described on the assumption that a previous image without halation will be displayed instead of the latest image captured during the PDT when it has halation like in the first embodiment. However, the therapeutic efficiency of the PDT may be improved by providing the PDT preparation mode also where the PDT and the ordinary light imaging are alternately carried out.

In the embodiment shown in Figs. 14 to 16, the PDT preparation mode may be introduced before the PDT so that the operator may execute the PDT more efficiently. The following embodiment is intended to visualize the therapeutic efficiency of the PDT during the execution of the PDT.

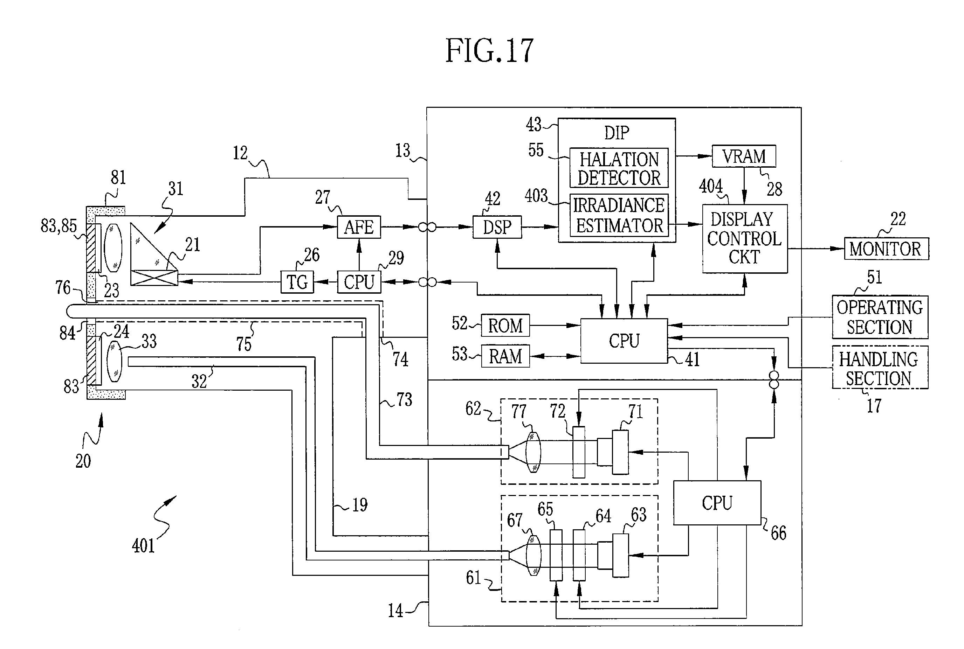

As shown in Fig.17, an electronic endoscope system 401 fundamentally has the same structure and functions as the electronic endoscope system 301. However, an irradiance estimator 403 of the electronic endoscope system 401 works not only during the PDT preparation mode but also during the PDT, so as to calculate total amounts of irradiation with the therapeutic light from the sequentially captured PDT images. Specifically, the irradiance estimator 403 sequentially adds up the pixel levels of the corresponding pixels of the successive PDT images, to calculate a cumulative value of each individual pixel included in a range of interest (ROI) that may be designated by the operator. Because the therapeutic light usually has a very high intensity in comparison with the ordinary light, the cumulative value of each pixel generally represents the integrated amount of irradiation at a corresponding point with the therapeutic light. Therefore, the cumulative values of the respective pixels, calculated by the irradiance estimator 403, will be referred to as the integrated amounts of irradiation of the corresponding points in the ROI with the therapeutic light.

In order to calculate the integrated amounts of irradiation, the irradiance estimator 403 adds up the pixel levels of each pixel regardless of whether the captured PDT image is with halation or without. The irradiance estimator 403 also continues to calculate the integrated amounts of irradiation from the start of projection of the therapeutic light to the end of the PDT. When the operator interrupts projection of the therapeutic light by stepping off the treadle in the middle of the PDT, the irradiance estimator 403 stops adding up the pixel levels. However, when the operator steps on the treadle again to restart projecting the therapeutic light, the irradiance estimator 403 also restarts adding up the pixel levels to those pixel levels which have been calculated before the interruption of the therapeutic light. In addition, the irradiance estimator 403 successively produces a mapped image that is a color map or a gray scale map showing the distribution of current irradiances of an aimed surface by the therapeutic light; the color or density of respective pixels of the mapped image changes with the integrated amounts of irradiation at the corresponding surface points according to a predetermined color scale or gray scale. Thus the bitmapped image shows the effect of irradiation with the therapeutic light. The mapped images are sequentially fed to a display control circuit 404.

The display control circuit 404 controls a monitor 22 to display histograms 311 and 312 in the PDT preparation mode in the same way as the display control circuit 304 of the embodiment of Fig.14. The display control circuit 404 also selects the PDT image without halation sequentially from a VRAM 28 and superimposes the mapped image, which is sequentially fed from the irradiance estimator 403, onto the PDT image, to display the PDT image with the mapped image superimposed thereon. Specifically, the mapped image is superimposed on the ROI 313 in the displayed image.

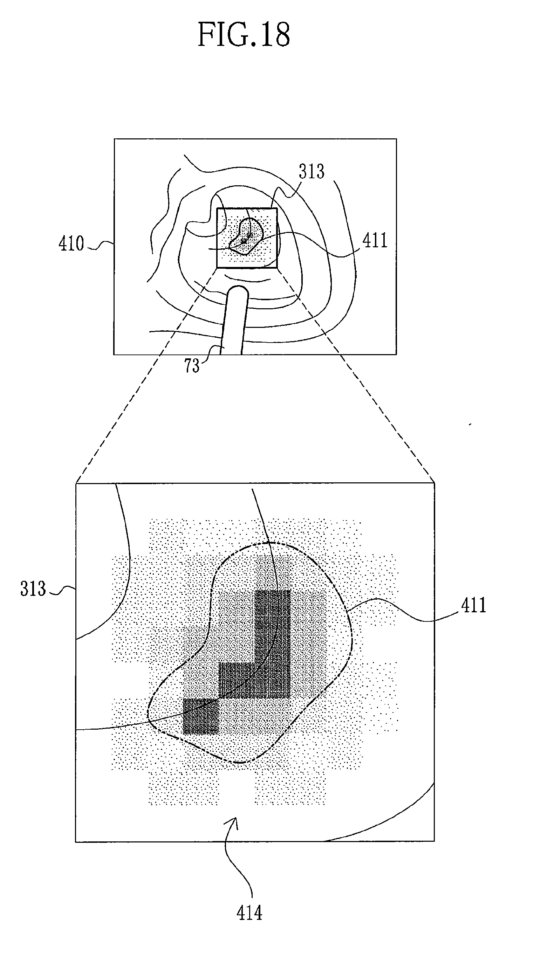

As shown for example in Fig.18, a PDT image 410 having a mapped image 414 superimposed on a range of interest (ROI) 313 is displayed on the monitor 22. The mapped image 414 superimposed on the PDT image 410 shows the irradiance distribution, i.e. the distribution of integrated amounts of irradiation in the ROI 313. Since the color or density of respective pixels of the mapped image changes with the integrated amounts of irradiation of the corresponding points, the operator can confirm how much a tumor 411 in the ROI 313 has been irradiated with the therapeutic light. For example, when the entire range of interest 313 gets a predetermined color or maximum density, it indicates that the tumor 411 has been sufficiently irradiated with the therapeutic light. Thus the operator can adjust the position and orientation of the tip 20 and the treatment probe 73 so that the tumor 411 gets quickly and uniformly colored in the predetermined color. Note that an enlarged view of the ROI 313 may be produced and displayed on the monitor 22 in addition to the PDT image 410 with the superimposed mapped image, like as shown in Fig.18. It may also be possible to provide an enlarged view mode where the enlarged view of the ROI 313 is displayed fully on the screen of the monitor 22.

As described above, the electronic endoscope system 401 produces a mapped image that represents cumulative values of the respective pixels of successive PDT images and superimposes the mapped image on the PDT image to be displayed on the monitor 22. From the superimposed mapped image, the operator can see the progress of the treatment during the PDT.

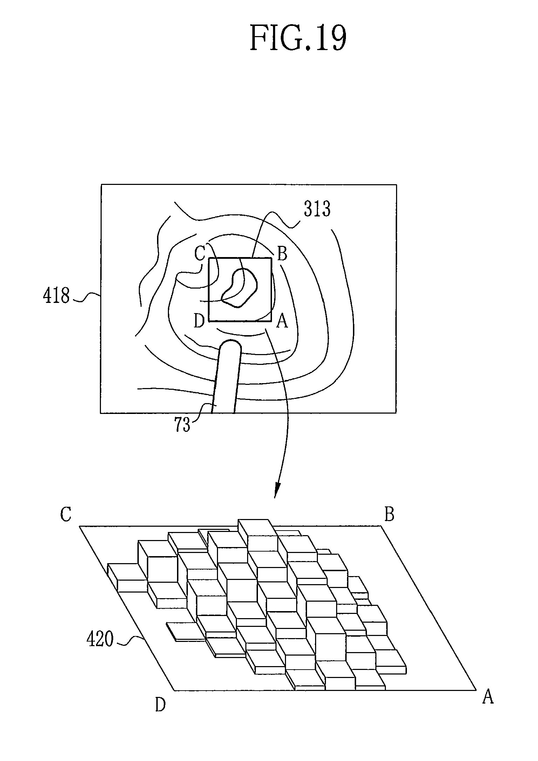

The irradiance distribution of the therapeutic light may be displayed in other ways than the above described color map or gray scale. For example, as shown in Fig.19, an image of a 3D histogram 420 may be produced instead of the 2D mapped image 414, to indicate the integrated amounts of irradiation by the therapeutic light with respect to the respective pixels of the roi 313. In this case, the 3D histogram image 420 may preferably be displayed in parallel to a corresponding PDT image 418 on the monitor 22. The 3D histogram image 420 may also be displayed in addition to the PDT image 410 that has the 2D mapped image 414 superimposed on the ROI 313.

In the above embodiment, the mapped image shows the irradiance distribution of the therapeutic light with respect to the pixels in the designated range of interest (ROI). It is alternatively possible to produce a mapped image that shows the irradiance distribution with respect to all pixels of the PDT image, and superimpose the mapped image on the entire area of the displayed PDT image.

Although the electronic endoscope system 401 is configured to display a previous image without halation in place of the latest PDT image if it has been captured with halation, the irradiance distribution of the therapeutic light may also preferably be displayed in the same way as described above in such a case where the therapeutic light and the ordinary light are alternately projected into the subject body, like as shown in Fig.9B, if the endoscopic imaging is carried out under the therapeutic light. The PDT preparation mode may be omitted from the electronic endoscope system 401.

More preferably, in addition to the mapped image or the stereoscopic histogram image 420, an edge of halation zone or overexposed part of a PDT image or a projection spot of the therapeutic light may be displayed in the PDT image on the monitor, like as shown in Figs.12 and 13 with respect to the electronic endoscope system 121 of Fig.11.

In the above embodiments, the DIP 43 functions as the halation detector. Alternatively, the DSP 42 or another component may function as the halation detector, or a specific circuit for the halation detector may be provided separately from the DIP 43 or the DSP 42. The threshold value for comparing respective pixel levels of a captured image to detect overexposed pixels, and the threshold value for use in judging by the number of overexposed pixels whether halation occurs in the captured image may be determined appropriately case by case.

In the above embodiments, an image without halation is forcibly captured during the PDT by closing the stop aperture 91 to block the therapeutic light the next frame when a predetermined number of images, e.g. four images, have been successively captured with halation. However, the present invention is not limited to this configuration. For example, the size of the stop aperture 91 may be reduced to lower the intensity of the therapeutic light in order to capture an image without halation during the PDT.

The VRAM 28 can store five image frames in the above embodiment, but the memory capacity of the VRAM 28 is not to be limited to five images but may be modified appropriately insofar as it can store more than one image frame. However, if the VRAM 28 has a small capacity, e.g. for two frames, the therapeutic light should probably be interrupted more frequently and hence the total treatment time could not effectively be reduced. In contrast, if the capacity of the VRAM 28 available for storing the PDT images is too large, the frequency of forcible imaging of an image without halation would get so low that a previous image without halation might be displayed for such a long time that the operator cannot precisely observe the present condition of the interior of the subject body. From these aspects, the VRAM 28 preferably has a capacity for five to fifteen frames for the sake of visualizing the interior of the subject body or the tumor site as clearly and instantaneously as possible.

In the above embodiments, the electronic endoscope systems use the CCD image sensor 21 as the imaging device, but the image sensor may be of CMOS type. It is possible to use an imaging device that captures three primary color image signals in a frame sequential fashion while changing the color of the illumination light between red, green and blue. However, in order to save the treatment time, it is preferable to use such an imaging device that is provided with color filters, e.g. RGB color filters, in correspondence with the respective sensor pixels to capture color image signals concurrently from the imaging device.

In the above embodiments, the pulse interval of the therapeutic light projection coincides with the driving interval of the CCD 21, i.e. the imaging interval, so that the therapeutic light is projected at the center of each frame (see Fig.9). However, the driving interval may not necessarily coincide with the pulse interval of the therapeutic light. In a case where the driving interval does not coincide with the pulse interval of the therapeutic light, the therapeutic light will not be projected in some frames that occur at regular intervals, so the imaging device will periodically capture images without the therapeutic light i.e. without halation even during the PDT mode.

The therapeutic light may not necessarily be projected as pulsed laser beam, but may be projected continuously during the PDT.

The photosensitizer used for the PDD and PDT may be any photosensitive substance or chemical insofar as it deposits in a target site to be treated, such as tumor cells, and emits fluorescence responding to the exciting light, and develops a therapeutic effect on the target site when irradiated with the therapeutic light. The exciting light for the PDD and the therapeutic light for the PDT may be set at appropriate wavelengths according to the adopted photosensitizer. Where many kinds of photosensitizers having sensitivities to different wavelength bands are applied, corresponding light sources may be provided.

In the above embodiments, the illumination light unit 61 can project either the ordinary light or the exciting light using the wavelength filter 65. In another embodiment, the wavelength filter 65 may have a function to limit the ordinary light from the light source 63 to a specific narrowband ray, hereinafter called a special ray, in addition to a function to transmit only the exciting light among the components of the ordinary light. In that case, the wavelength filter 65 may be a rotary disk having a sector for transmitting the ordinary light, a sector for transmitting the exciting light, and a sector for transmitting the special ray. The special ray may include a ray of around 450 nm, 500 nm, 550 nm, 600 nm, and 780 nm.

Imaging under the special ray of around 450 nm is suitable for endoscopy of microstructures on the surface of the target site, such as superficial blood vessels or pit patterns. Under the illumination of around 500 nm ray, micro textures of the target site, such as concavity and convexity in the target site, can be well visualized. The ray around 550 nm is highly absorbed in hemoglobin and hence suitable for endoscopy of micro vessels and rubor or redness. The ray around 650 nm is suitable as illumination light for observing thickenings. In order to observe the deep-layer blood vessels, intravenous injection of a fluorescent material such as indocyanine green (ICG) and illumination with rays of around 780 nm will provide clear images of the deep-layer blood vessels.

Although the exciting light for the PDD is produced from the ordinary light through the wavelength filter 65 in the above embodiments, a specific light source for emitting the exciting light for the PDD may be provided separately from the light source 63 for the ordinary light. In that case, the ordinary light and the exciting light may be alternatively introduced into the light guide 32 using a switching optical system that may include a mirror or the like.

Although the light source 63 may be a halogen lamp or a xenon lamp like in the above embodiment, the light source 63 may also be constituted of LEDs or LDs. In the latter case, the LEDs or LDs preferably emit rays of different wavelength bands so that switching between the ordinary light and the exciting light may be done by turning some of these LEDs or LDs on and off. The ordinary light may be produced using a blue laser light source and a fluorescent member that emits fluorescent light in a range from green to red when irradiated with the blue laser. Moreover, a light source provided for other use may be applied to the illumination for the endoscopy.

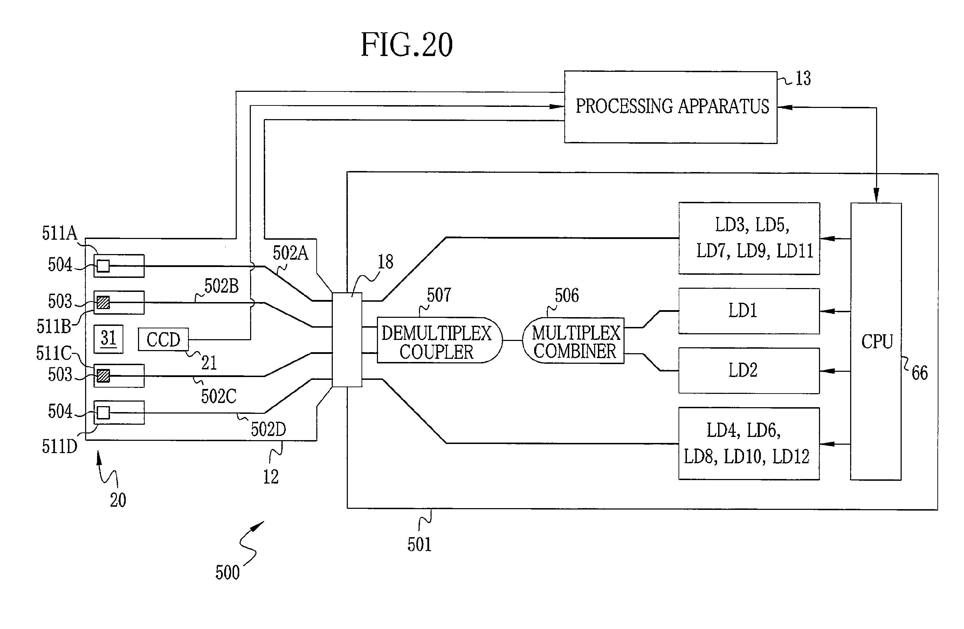

For example, as shown in Fig.20, a light source apparatus 501 of an electronic endoscope system 500 includes multiple lasers LD1 to LD12 that can emit rays of different center wavelengths from each other. Under the control of a CPU 66, the lasers LD1 to LD12 may individually change its light volume and may emit the rays independently or simultaneously. The timing of emission from each laser diode as well as the ratio of luminous intensity between the respective laser diodes may be appropriately modified, so that it is possible to change the spectrum of the illumination light projected from lightening windows individually.

The laser LD1 is a light source that emits light having a center wavelength of 405 nm and is used for endoscopy under a narrowband illumination light. The laser LD2 is a light source that emits a ray having a center wavelength of 445 nm and is used for producing white light or ordinary light in combination with a fluorescent member as a wavelength converter. The lasers LD3 and LD4 emit a ray having a center wavelength of 405 nm and are used for observing fluorescence like in the PDD. The lasers LD5 and LD6 emit a ray having a center wavelength of 472 nm and are used for extracting information about blood oxygen saturation and blood vessel depth. The lasers LD7 and LD8 emit a ray having a center wavelength of 665 nm and are used for the PDT. The lasers LD9 and LD10 emit a ray having a center wavelength of 785 nm and are used for observing infrared rays emitted from the indocyanine green injected in the blood vessels. The lasers LD11 and LD12 emit light having a center wavelength of 375 nm and are used for endoscopy using fluorescence from luciferase. Note that the rays from the respective lasers may have a range of ±10 nm around each center wavelength.

The laser light from each of the lasers LD1 to LD12 is introduced into optical fibers 502A to 502D through a not-shown condenser lens. Specifically, the laser light from LD1 and LD2 is conducted through the optical fibers 502B and 502C to phosphors 503 that are disposed in a tip 20 of an endoscope 12. The laser light from LD3 to LD12 is conducted through the optical fibers 502A and 502D to light diffusers 504, which project the laser light as illumination light, exiting light or therapeutic light into a subject body.

The laser light from LD1 and LD2 is combined at a combiner 506 into a bundle of light and thereafter branched at a coupler 507 into two bundles, which are transmitted to a connector 18 and then conducted respectively through the optical fibers 502B and 502C. Thus the variations in emission wavelength between individual lasers as well as speckles are reduced so that uniform laser light will be conducted through the optical fibers 502B and 502C. However, it is possible to omit the combiner 506 and the coupler 507 and transmit the laser light from the lasers LD1 and LD2 simply to the connector 18.

The optical fiber 502B and the phosphor 503 constitute a light projection unit 511B, whereas the optical fiber 502C and the phosphor 503 constitute a light projection unit 511C. The optical fiber 502A and the light diffuser 504 constitute a light projection unit 511A, whereas the optical fiber 502D and the light diffuser 504 constitute a light projection unit 511D.

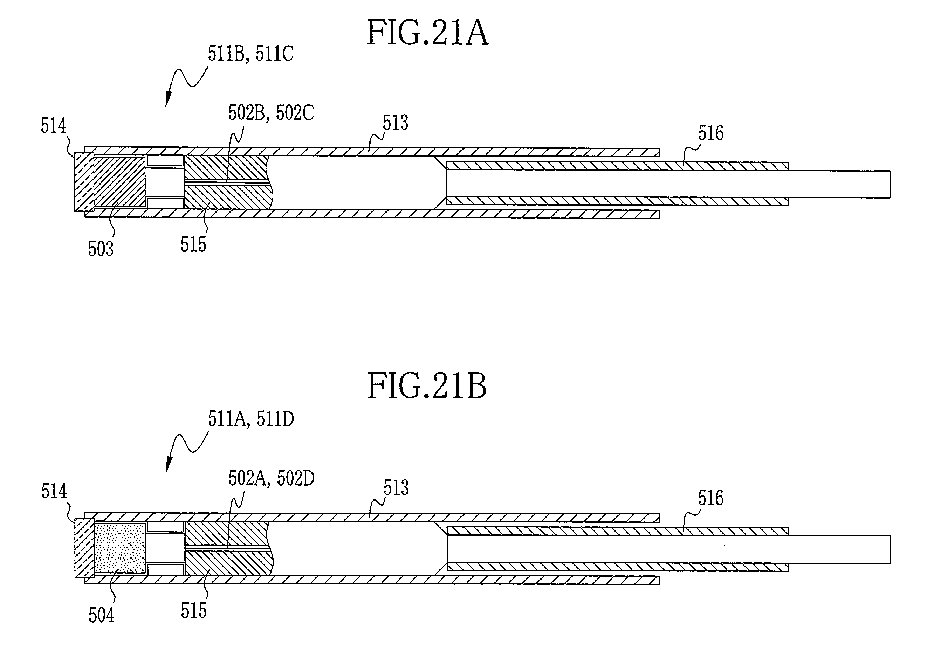

As shown in detail in Fig.21A, the light projection units 511B and 511C have an identical structure to each other, consisting of the phosphor 503, a sleeve 513 covering the periphery of the phosphor 503, a protection glass or lighting window 514 closing a distal end of the sleeve 513, and a ferrule 515 holding the optical fiber 502B or 502C in an axial center inside the sleeve 513. In addition, a flexible sleeve 516 is inserted into between the sleeve 513 and the optical fiber 502B or 502C in a section extending proximately from a proximal end of the ferrule 515.

The phosphors 503 of the light projection units 511B and 511C contain such a fluorescent substance that is excited to emit green to yellow fluorescence as it absorbs part of blue laser light from the laser LD2, e.g. YAG phosphor or BAM (BaMgAl₁₀O₁₇) phosphor. Consequently, the green to yellow light from the blue-laser excited phosphor 503 is mixed with remaining part of the blue laser light that is not absorbed in the phosphor 503 to produce the white or pseudo-white ordinary light.

As shown in Fig. 21B, the light projection units 511A and 511B have an identical structure to each other, which is equal to the structure of the light projection units 511B and 511C except that the light diffuser 504 is disposed in place of the phosphor 503 and the light is conducted through the optical fiber 502A or 502D.

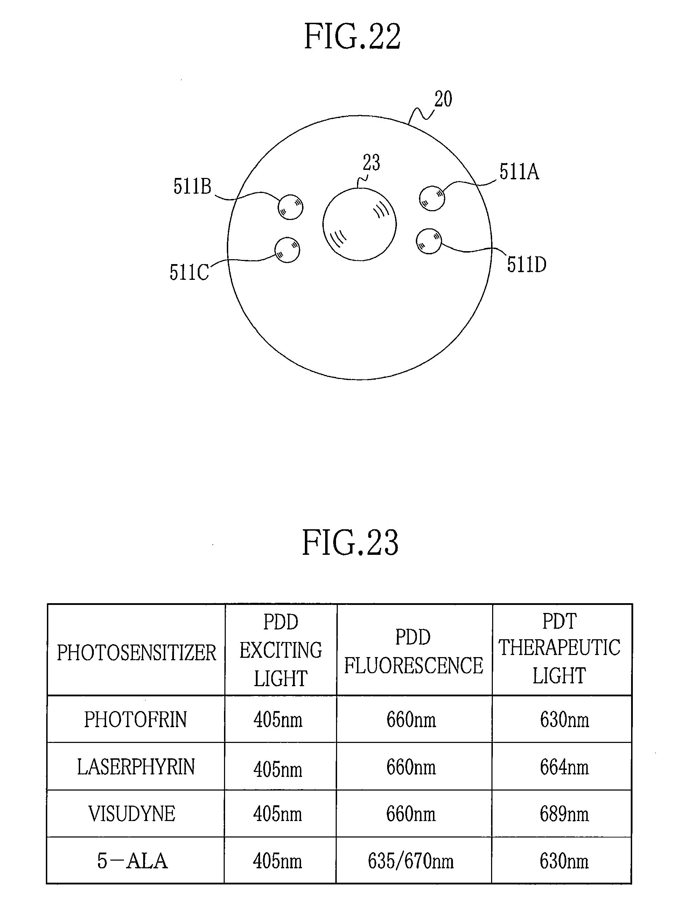

As shown in Fig.22, the light projection units 511A and 511D are disposed as a pair on one side of an objective lens system 31 in a face of the endoscope tip 20, and the light projection units 511B and 511C are disposed as another pair on the other side of the objective lens system 31. The light projection units 511B and 511C project white light or ordinary light for example when the blue laser light from the laser LD2 is introduced into the optical fibers 502B and 502C. The light projection units 511A and 511D selectively project the exciting light for the PDD or the therapeutic light for the PDT. The light projection units 511A and 511D project the exciting light for example when the laser light of around 405 nm is introduced from the lasers LD3 and LD4 into the optical fibers 502A and 502D. On the other hand, the light projection units 511A and 511D project the therapeutic light for example when the laser light of around 665 nm is introduced from the lasers LD7 and LD8 into the optical fibers 502A and 502D.

Referring to Fig.23, center wavelengths of the exciting light for the PDD, the fluorescence reactive to the exciting light, and the therapeutic light for the PDT are listed in a table with respect to available photosensitizers or fluorescent drugs. To any of these fluorescent drugs, including Photofrin, Laserphyrin, Visudyne and 5-ALA (aminolevulic acid), any laser light having a center wavelength of 350 nm to 450 nm may be applicable as the exciting light for the PDD, and the laser light having the center wavelength of 405 nm is the most preferable. Note that 5-ALA is accumulated as protoporphyrin IX in a lesion and the wavelength ratio of the fluorescence from this drug changes depending upon the progression of the lesion.

It should be understood that the embodiments of the present invention have been disclosed for illustrative purposes only. Those skilled in the art will appreciate that various modifications, additions and substitutions are possible without departing from the scope and spirit of the invention as disclosed in the accompanying claims.

## Claims

1. An electronic endoscope system having
a white light projecting device (61, 511B, 511C) for projecting white light into an interior of a subject body;
a therapeutic light projecting device (62, LD7, LD8) for projecting therapeutic light onto a therapeutic target site (103, 322) to be treated inside the subject body, said therapeutic light giving a therapeutic effect on the target site by acting on a photosensitive substance that has been accumulated in the target site; and
an imaging device (21) for imaging the interior of the subject body through photoelectric conversion of light received from the interior of the subject body,
said electronic endoscope system **characterized by** comprising:
a light volume adjusting device (72, 66) for adjusting the volume of said therapeutic light to such a low intensity that no halation can occur in an image captured by said imaging device while said white light and said low intensity therapeutic light are being projected into the interior of the subject body;
an extracting device (303) for extracting from said captured image data about the position and power of irradiation by said low intensity therapeutic light inside the subject body; and
a display control device (304) for controlling a display device (22) to display the data extracted by said extracting device in combination with said image.

2. The electronic endoscope system as recited in claim 1, further comprising:
a range designating device (13) for designating a range of interest (313) in said captured image, wherein
said extracting device (303) extracts the data about the distribution of irradiance of said low intensity therapeutic light along vertical and horizontal axes of said image, said axes intersecting at a center of the range of interest.

3. The electronic endoscope system as recited in claim 1 or 2, wherein said extracting device (303) extracts said data based on pixel levels of one color among pixel levels of said image, said one color corresponding to a wavelength band of said therapeutic light.

4. The electronic endoscope system as recited in any of claims 1 to 3, wherein said display control device (304) produces histograms (311, 312) from said extracted data and controls said display device (22) to display the histograms with said image.

5. The electronic endoscope system as recited in any of claims 1 to 4, further comprising:
an exciting light projecting device (63, 65, LD3, LD4) for projecting exciting light into the interior of the subject body, said exciting light causing said photosensitive substance to emit fluorescence;
an identifying device (13) for identifying the target site based on a fluorescent light image (102) captured by imaging the fluorescent light that is emitted from said photosensitive substance upon said exciting light being projected into the interior of the subject body;
said range designating device (13) automatically designates the range of interest to include the target site (322) as identified by said identifying device.

6. The electronic endoscope system as recited in any of claims 1 to 5, further comprising:
a halation detecting device (55) for detecting whether an image frame has halation or not when said image frame has been captured by said imaging device while said therapeutic light is being projected concurrently with said white light into the subject body; and
a memory device (28) for storing a plural number of image frames captured by said imaging device (21), wherein
when said halation detecting device detects that the latest image frame captured by said imaging device has halation, said display control device selects an image frame without halation from among image frames stored in said memory device, to display the selected image frame instead of the latest image frame on said display device.

7. The electronic endoscope system as recited in claim 6, wherein said halation detecting device (55) determines that one image frame has halation when the number of those pixels having higher levels than a predetermined threshold value is above a predetermined number among pixels constituting said one image frame, and that said one image frame does not have halation when the number of those pixels having higher levels than the predetermined threshold value is not more than the predetermined number.

8. The electronic endoscope system as recited in any of claim 6 or 7, further comprising a control device (41) for controlling said light volume adjusting device (72, 66) to lower the intensity of said therapeutic light to forcibly capture an image frame without halation.

9. The electronic endoscope system as recited in any of claim 8, wherein said memory device (28) temporarily stores a constant number of image frames in the order captured, and said control device (41) controls said light volume adjusting device (72) to lower the intensity of said therapeutic light before all image frames stored in said memory device (28) have halation.

10. The electronic endoscope system as recited in claim 9, wherein said control device (41) controls said light volume adjusting device (72, 66) to lower the intensity of said therapeutic light in the next frame when a predetermined number of successive image frames have been captured with halation, said predetermined number being smaller than said constant number.
